# EUROPÄISCHE PATENTSCHRIFT

(11) **EP 2 089 390 B1**
(45) Veröffentlichungstag und Bekanntmachung des Hinweises auf die Patenterteilung: **27.07.2011**
(21) Anmeldenummer: 07821820.3
(22) Anmeldetag: 25.10.2007
(51) Int. Cl.: C07D 471/06, C09B 5/62, C07D 221/18

(54) **HEPTARYLEN-UND OCTARYLENTETRACARBONSÄUREDIIMIDE UND DEREN HERSTELLUNG**
HEPTARYLENE AND OCTARYLENE TETRACARBOXYLIC ACID DIIMIDES, AND PRODUCTION THEREOF
DIIMIDES D'ACIDE HEPTARYLÈNE-TÉTRACARBOXYLIQUE ET D'ACIDE OCTARYLÈNE-TÉTRACARBOXYLIQUE ET LEUR PRODUCTION

(30) Priorität: 02.11.2006 EP 06123380
(43) Veröffentlichungstag der Anmeldung: 19.08.2009
(73) Patentinhaber: BASF SE, 67056 Ludwigshafen (DE); Max-Planck-Gesellschaft zur Förderung der Wissenschaften e.V., 80539 München (DE)
(72) Erfinder: QU, Jianqiang, 67061 Ludwigshafen (DE); PSCHIRER, Neil Gregory, 55116 Mainz (DE); KÖNEMANN, Martin, 68163 Mannheim (DE); MÜLLEN, Klaus, 50939 Köln (DE); AVLASEVIC, Yuri, 55122 Mainz (DE)
(86) Internationale Anmeldenummer: PCT/EP2007/061457
(87) Internationale Veröffentlichungsnummer: WO 2008/052927

(56) Entgegenhaltungen:
- WO-A-02/068538
- WO-A-2004/026965
- WO-A-2006/111511

## Beschreibung

Die vorliegende Erfindung betrifft Heptarylen- und Octarylentetracarbonsäurediimide sowie deren Herstellung und Verwendung.

Rylentetracarbonsäurediimide sind bekanntermaßen aufgrund ihrer starken Absorption im Nahinfrarot (NIR-)-Bereich des elektromagnetischen Spektrums von besonderem anwendungstechnischen Interesse.

So wird zum Beispiel in WO-A 02/77081 der Einsatz von Quaterrylentetracarbonsäurediimiden als Infrarotabsorber für den Wärmeschutz in Glaslaminaten beschrieben.

Pentarylen- und Hexarylenderivate, die unsubstituiert sind oder einen geringen Substitutionsgrad aufweisen, sind von N. G. Pschirer et al., Angew. Chem. Int. Ed. 45 (2006), 1401-1404 beschrieben.

Ähnliche Pentarylen- und Hexarylenderivate sind auch in DE-A 10 2005 018241 beschrieben. Thermochrome Terrylen- und Quaterylen-Farbstoffe wurden in WO-A 02/068538 beschrieben. WO-A 2004/026965 beschreibt Anthrachinonrylen-Derivate und deren Herstellung sowie Verwendung als Farbstoffe.

Trotz der bereits beschriebenen Pentarylen- und Hexarylenderivate sowie deren Verwendung im Zusammenhang mit deren Absorptionsvermögen im NIR besteht ein Bedarf an weiteren höheren, insbesondere hochsubstituierten, Derivaten sowie an Verfahren zu deren Herstellung.

Eine Aufgabe der vorliegenden Erfindung ist somit die Bereitstellung solcher Derivate und Verfahren zu deren Herstellung, die insbesondere aufgrund ihres Absorptionsvermögens von besonderem Interesse sind und aufgrund möglichst einfacher Herstellverfahren trotz ihres vergleichsweise hohen Molekulargewichts und gegebenenfalls hohen Substitutionsgrades leicht herstellbar sein sollen.

Diese Aufgabe wird gelöst durch ein Verfahren zur Herstellung von Heptarylen- und Octarylentetracarbonsäuredümiden, die Schritte enthaltend
(a) Kupplung mindestens einer Quaterrylenverbindung der Formel (II) 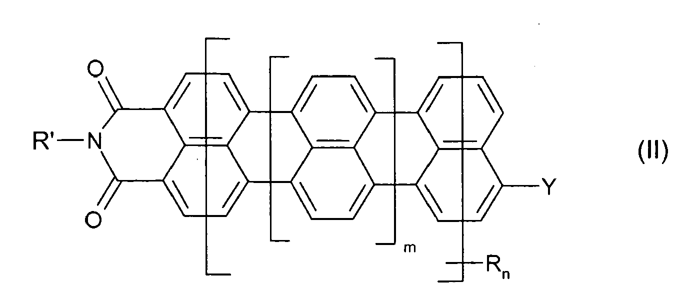 mit mindestens einer Verbindung der Formel (III) 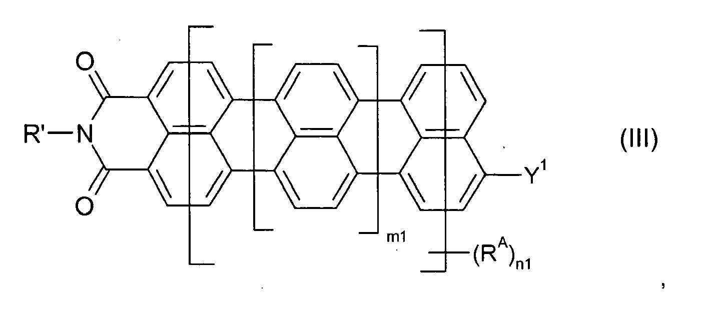 wobei
   Y, Y¹ beide Halogen sind oder ein Rest von Y, Y¹ Halogen und der andere B(OR")₂ ist;
   Jedes R, R^{A} unabhängig voneinander gleiche oder verschiedene der folgenden Reste sind:
   Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
      (i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder - SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ ,-POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
      (ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder - SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder - SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², - SO₃R², -PR²R³ und/oder -POR²R³;
      (iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, - CONR²R³, -SO₂NR²R³, -COOR², -SO₃R², -PR²R³ und/oder -POR²R³ substituiert sein kann;
      (iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
      (v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SONR²R³, - COOR², -SO₃R², -PR²R³ und/oder -POR²R³;
   jedes R' jeweils unabhängig Wasserstoff;
   C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder - SO₂- unterbrochen sein kann und das ein- oder mehrfach durch die als Substituenten für die Reste R genannten Reste (ii), (iii), (iv) und/oder (v) substituiert sein kann;
   C₃-C₈-Cycloalkyl, an das weitere gesättigte oder ungesättigte 5- bis 7-gliedrige Ringe, deren Kohlenstoffgerüst durch eine oder mehrere grupperungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem durch die als Substituenten für die Reste R genannten Reste (i), (ii), (iii), (iv) und/oder (v) substituiert sein kann; oder
   Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem substituiert sein kann durch die als Substituenten für die Reste R genannten Reste (i), (ii), (iii), (iv), (v), Aryl- und/oder Hetarylazo, das jeweils durch C₁-C₁₀-Alkyl, C₁-C₆-Alkoxy und/oder Cyano substituiert sein kann, ist;
   jedes R" unabhängig voneinander Wasserstoff, C₁-C₃₀-Alkyl, C₅-C₈-Cycloalkyl, Aryl oder Hetaryl ist oder miteinander verbunden unter Ausbildung eines die beiden Sauerstoffatome sowie das Boratom enthaltenden 5- bis 7-gliedrigen Rings, an den ungesättigte oder gesättigte Ringe anneliert sein können und der an den Kohlenstoffatomen durch bis zu 4 C₁-C₃₀-Alkyl-, C₅-C₈-Cycloalkyl-, Aryl- oder Hetarylgruppen substituiert sein kann, sind;
   R¹ Wasserstoff oder C₁-C₁₈-Alkyl, wobei die Reste R¹ gleich oder verschieden sein können, wenn sie mehrfach auftreten, ist;
   R², R³ unabhängig voneinander Wasserstoff;
   C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
   Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, sind;
   und wobei m, m1, n, n1 mindestens eine der folgenden Bedingungen erfüllen:
      m = 2, m1 = 1 und n, n1 sind ganze Zahlen, deren Summe eine Zahl von 0 bis 12 ergibt;
      m = 2, m1 = 2 und n, n1 sind ganze Zahlen, deren Summe eine Zahl von 0 bis 16 ergibt;
(b) Cyclodehydrierung des in Schritt (a) erhaltenen Umsetzungsproduktes zu einer Rylenverbindung der allgemeinen Formel (I) 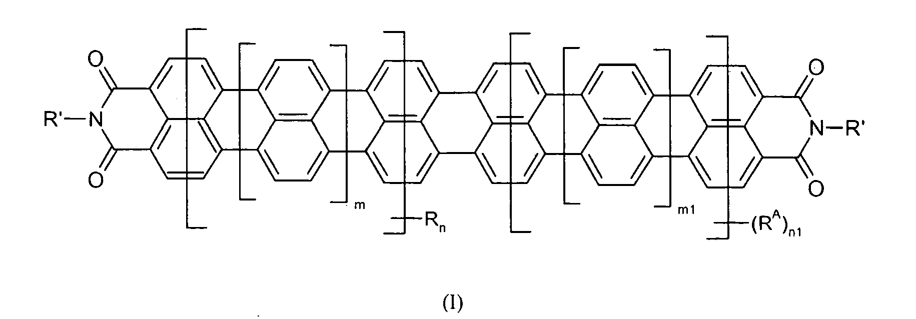 oder deren Mischungen.

Für das erfindungsgemäße Verfahren erfüllen m, m1, n, n1 mindestens eine der folgenden Bedingungen erfüllen.

Zur Herstellung von Heptarylentetracarbonsäurediimiden werden ein entsprechendes Quaterrylen und ein entsprechendes Terylen (m = 1, m1 = 1) umgesetzt, wobei n, n1 ganze Zahlen sind, deren Summe eine Zahl von 0 bis 12, also 0, 1, 2, 3, 4, 5, 6, 7, 8, 9, 10, 11 oder 12, vorzugsweise 4, 5, 6, 7, 8, 9 oder 10, insbesondere 5, 6, 7 oder 8, ergibt. Beispiele für n + n1 sind 8 + 0, 7 + 0, 6 + 0, 5 + 0, 4 + 0, 3 + 0, 2 + 0, 1 + 0, 0 + 0, 8 + 1, 7 + 1, 6 + 1, 5 + 1, 4 + 1, 3 + 1, 2 + 1, 1 + 1, 0 + 1, 8 + 2, 7 + 2, 6 + 2, 5 + 2, 4 + 2, 3 + 2, 2 + 2, 1 + 2, 0 + 2, 8 + 3, 7 + 3, 6 + 3, 5 + 3, 4 + 3, 3 + 3, 2 + 3, 1 + 3, 0 + 3, 8 + 4, 7 + 4, 6 + 4. 5 + 4, 4 + 4, 3 + 4, 2 + 4, 1 + 4, 0 + 4, 7 + 5, 6 + 5, 5 + 5, 4 + 5, 3 + 5, 2 + 5, 1 + 5, 0 + 5, 6 + 6, 5 + 6, 4 + 6, 3 + 6, 2 + 6, 1 + 6, 0 + 6. Bevorzugt für n + n1 sind 6 + 4, 5 + 4, 4 + 4, 3 + 4, 6 + 3, 5 + 3, 4 + 3, 3 + 3, 6 + 2, 5 + 2, 4 + 2, 3 + 2, 2 + 2.

Zur Herstellung von Octarylentetracarbonsäurediimiden werden zwei entsprechende Quaterrylene (m = 2, m1 = 2) umgesetzt, wobei n, n1 ganze Zahlen sind, deren Summe eine Zahl von 0 bis 16, also 0, 1, 2, 3, 4, 5, 6, 7, 8, 9, 10, 11, 12, 13, 14, 15 oder 16, vorzugsweise 4, 5, 6, 7, 8, 9 oder 10, mehr bevorzugt 4, 5, 6, 7 oder 8 insbesondere 5, 6, 7 oder 8 ergibt. Beispiele für n + n1 sind 8 + 0, 7 + 0, 6 + 0, 5 + 0, 4 + 0, 3 + 0, 2 + 0, 1 + 0, 0 + 0, 8 + 1, 7 + 1, 6 + 1, 5 + 1, 4 + 1, 3 + 1, 2 + 1, 1 + 1, 0 + 1, 8 + 2, 7 + 2, 6 + 2, 5 + 2, 4 + 2, 3 + 2, 2 + 2, 1 + 2, 0 + 2, 8 + 3, 7 + 3, 6 + 3, 5+ 3, 4 + 3, 3 + 3, 2 + 3, 1 + 3, 0 + 3. 8 + 4, 7 + 4, 6 + 4, 5 + 4, 4 + 4, 3 + 4, 2 + 4, 1 + 4, 0 + 4, 8 + 5, 7 + 5, 6 + 5, 5 + 5, 4 + 5, 3 + 5, 2 + 5, 1 + 5, 0 + 5, 8 + 6, 7 + 6, 6 + 6, 5 + 6, 4 + 6, 3 + 6, 2 + 6, 1 + 6, 0 + 6, 8 + 7, 7 + 7, 6 + 7, 5 + 7, 4 + 7, 3 + 7, 2 + 7, 1 + 7, 0 + 7, 8 + 8, 7 + 8, 6 + 8, 5 + 8, 4 + 8, 3 + 8, 2 + 8, 1 + 8, 0 + 8. Bevorzugt für n + n1 sind 6 + 6, 6 + 5, 6 + 4, 6 + 3, 5 + 6, 5 + 5, 5 + 4, 5 + 3, 4 + 6, 4 + 5, 4 + 4, 4 + 3, 3 + 6, 3 + 5, 3 + 4, 3 + 3, 2 + 2.

Weiterhin wird die Aufgabe gelöst durch Heptarylentetracarbonsäurediimide der allgemeinen Formel (I) oder Mischungen davon, wobei n und n1 ganze Zahlen sind, deren Summe eine Zahl von 0 bis 12, also 0, 1, 2, 3, 4, 5, 6, 7, 8, 9, 10, 11 oder 12, vorzugsweise 4, 5, 6, 7, 8, 9 oder 10, insbesondere 5, 6, 7 oder 8, ergibt und R, R^{A}, R', m, m1 die oben angegebene Bedeutung besitzen. Beispiele für n + n1 sind 8 + 0, 7 + 0, 6 + 0, 5 + 0, 4 + 0, 3 + 0, 2 + 0, 1 + 0, 0 + 0, 8 + 1, 7 + 1, 6 + 1, 5 + 1, 4 + 1, 3 + 1, 2 + 1, 1 + 1, 0 + 1, 8 + 2, 7 + 2, 6 + 2, 5 + 2, 4 + 2, 3 + 2, 2 + 2, 1 + 2, 0 + 2, 8 + 3, 7 + 3, 6 + 3, 5 + 3, 4 + 3, 3 + 3, 2 + 3, 1 +3, 0 + 3, 8 + 4, 7 + 4, 6 + 4, 5 + 4, 4 + 4, 3 + 4, 2 + 4, 1 + 4, 0 + 4, 7 + 5, 6 + 5, 5 + 5, 4 + 5, 3 + 5, 2 + 5, 1 + 5, 0 + 5, 6 + 6, 5 + 6, 4 + 6, 3 + 6, 2 + 6, 1 + 6, 0 + 6. Bevorzugt für n + n1 sind 6 + 4, 5 + 4, 4 + 4, 3 + 4, 6 + 3, 5 + 3, 4 + 3, 3 + 3, 6 + 2, 5 + 2, 4 + 2, 3 + 2, 2 + 2.

Die erfindungsgemäßen Heptarylenverbindungen können also mit Hilfe des erfindungsgemäßen Verfahrens durch Kupplung einer entsprechenden Quaterrylenverbindung mit der entsprechenden Terrylenverbindung erhalten werden. Die erfindungsgemäßen Heptarylenverbindungen können einen hohen Substitutionsgrad aufweisen. Die Summe der Substituenten R und R^{A} beträgt jedoch maximal 12.

Die Aufgabe wird weiterhin gelöst durch Octarylentetracarbonsäurediimide der allgemeinen Formel (I) oder Mischungen davon, wobei n und n1 ganze Zahlen sind, deren Summe 0 bis 16, also 0, 1, 2, 3, 4, 5, 6, 7, 8, 9, 10, 11, 12, 13, 14, 15 oder 16, vorzugsweise 4, 5, 6, 7, 8, 9 oder 10, mehr bevorzugt 4, 5, 6, 7 oder 8 insbesondere 5, 6, 7 oder 8 ergibt und R, R^{A}, R', m, m1 die oben angegebene Bedeutung besitzen. Beispiele für n + n1 sind 8 + 0, 7 + 0, 6 + 0, 5 + 0, 4 + 0, 3 + 0, 2 + 0, 1 + 0, 0 + 0, 8 + 1, 7 + 1, 6 + 1, 5 + 1, 4 + 1, 3 + 1, 2 + 1, 1 + 1, 0 + 1, 8 + 2, 7 + 2, 6 + 2, 5 + 2, 4 + 2, 3 + 2, 2 + 2, 1 + 2, 0 + 2, 8 + 3, 7 + 3, 6 + 3, 5+ 3, 4 + 3, 3 + 3, 2 + 3, 1 + 3, 0 + 3, 8 + 4, 7 + 4, 6 + 4, 5 + 4, 4 + 4, 3 + 4, 2 + 4, 1 + 4, 0 + 4, 8 + 5, 7 + 5, 6 + 5, 5 + 5, 4 + 5, 3 + 5, 2 + 5, 1 + 5, 0 + 5, 8 + 6, 7 + 6, 6 + 6, 5 + 6, 4 + 6, 3 + 6, 2 + 6, 1 + 6, 0 + 6,8 + 7, 7 + 7, 6 + 7, 5 + 7, 4 + 7, 3 + 7, 2 + 7, 1 + 7, 0 + 7,8 + 8, 7 + 8, 6 + 8, 5 + 8, 4 + 8, 3 + 8, 2 + 8, 1 + 8, 0 + 8, Bevorzugt für n + n1 sind 6 + 6, 6 + 5, 6 + 4, 6 + 3, 5 + 6, 5 + 5, 5 + 4, 5 + 3, 4 + 6, 4 + 5, 4 + 4, 4 + 3, 3 + 6, 3 + 5, 3 + 4, 3 + 3, 2 + 2.

Die erfindungsgemäßen Octarylenverbindungen können also mit Hilfe des erfindungsgemäßen Verfahrens durch Kupplung zweier entsprechenden Quaterrylenverbindungen erhalten werden. Die erfindungsgemäßen Octarylenverbindungen können einen hohen Substitutionsgrad aufweisen. Die Summe der Substituenten R und R^{A} beträgt jedoch maximal 16.

Die Ausgangsverbindungen gemäß der Formeln (II), (III), (IIIa) sind aus dem Stand der Technik bekannt oder können anhand literaturbekannter Synthesen analoger Verbindungen hergestellt werden. Insbesondere Terrylen- und Quaterrylenderivate, die als Ausgangsstoffe für das erfindungsgemäße Verfahren zur Herstellung von Heptarylen- und Octarylentetracarbonsäurediimiden dienen können, sind in der deutschen Patentanmeldung mit der Anmeldenummer 10 2005 021362 beschrieben. Darüber hinaus wird die Herstellung von Hexarylen- und Pentarylentetracarbonsäurediimiden in DE-A 10 2005 018241 beschrieben. Eine Herstellung der erfindungsgemäßen Imidverbindungen kann analog erfolgen.

Das erfindungsgemäße Verfahren zur Herstellung von Heptarylen- und Octrylentetracarbonsäurediimiden enthält als ersten Schritt (a) die Kupplung mindestens einer Quaterrylenverbindung der Formel (II) mit mindestens einer Verbindung der Formel (III), wobei die Verknüpfung der beiden Bausteine jeweils mit Hilfe der Gruppen Y und Y¹ erfolgt.

Dabei können Y und Y¹ Halogenide sein, durch die mit Hilfe einer katalytischen Kupplung die gewünschte Bindung der beiden aromatischen Bausteine ermöglicht wird. Ebenso ist es möglich, dass einer der Reste Y, Y¹ ein Halogenid und der andere eine Boronsäure oder eine ähnliche Verbindung der Formel B(OR")₂ sein kann. Eine Kupplung erfolgt dann über die sog. Suzuki-Reaktion. Vorzugsweise handelt es sich in beiden Fällen bei den Halogeniden um Bromid oder Chlorid.

Die Herstellung der erfindungsgemäßen Diimide mit Hilfe des erfindungsgemäßen Verfahrens erfolgt vorzugsweise in Gegenwart eines organischen Lösemittels, gewünschtenfalls im Gemisch mit Wasser, sowie eines Übergangsmetallkatalysators und einer Base, wobei wie oben ausgeführt wurde, einer der beiden Bausteine ein Boronäurederivat und der andere ein Halogenid darstellen kann.

Ein solches Boronsäurederivat ist beispielsweise durch Umsetzung des entsprechenden halogenierten Aromaten mit Hilfe von Diboranen der allgemeinen Formel (IV) (R"O)₂B-B(OR")₂ in Gegenwart eines aprotischen organischen Lösungsmittels, eines Übergangsmetallkatalysators und einer Base erhältlich.

Geeignete Diborane der allgemeinen Formel (IV) sind insbesondere Bis(1,2- und 1,3-diolato)diborane, Tetraalkoxydiborane, Tetracycloalkoxydiborane und Tetra(het)aryloxydiborane sowie deren Mischformen. Als Beispiele für diese Verbindungen seien genannt: Bis(pinacolato)diboran, Bis(1,2-benzodiolato)-diboran, Bis(2,2-dimethyl-1,3-propandiolato)diboran, Bis(1,1,3,3-tetramethyl-1,3-pro-pandiolato)diboran, Bis(4,5-pinandiolato)diboran, Bis(tetramethoxy)diboran, Bis(tetra-cyclopentoxy)diboran, Bis(tetraphenoxy)diboran und Bis(4-pyridiyloxy)diboran.

Bevorzugt sind Diborane der allgemeinen Formel (IV), bei denen die beiden an einem Boratom befindlichen Reste R" unter Ausbildung eines die beiden Sauerstoffatome sowie das Boratom enthaltenden Fünf- oder Sechsrings miteinander verbunden sind. An die gebildeten Fünf- oder Sechsringe können aromatische oder gesättigte, auch bicyclische, Ringe, mit 5 bis 7 C-Atomen als Ringglieder anneliert sein. Alle Ringe bzw. Ringsysteme können durch bis zu 4 C₁-C₃₀-Alkyl-, C₅-C₈-Cycloalkyl-, Aryl- und/oder Hetarylreste substituiert sein, vorzugsweise sind sie durch bis zu 4 C₁-C₄-Alkylreste substituiert. Beispiele für diese bevorzugten Diborane sind die bereits oben genannten Bis(1,2- und 1,3-diolato)-diborane, wobei Bis(pinacolato)diboran besonders bevorzugt ist.

Das Molverhältnis Diboran der allgemeinen Formel (IV) zum halogenierten Aromaten liegt dabei im Allgemeinen bei 0,8 : 1 bis 3 : 1, insbesondere bei 1,5 : 1 bis 2 : 1.

Als Lösungsmittel sind grundsätzlich alle unter den Reaktionsbedingungen gegen Basen stabilen aprotischen Lösungsmittel mit einem Siedepunkt oberhalb der gewählten Reaktionstemperatur geeignet, in denen sich die Edukte bei Reaktionstemperatur vollständig und die verwendeten Katalysatoren und Basen zumindest partiell lösen, so dass weitgehend homogene Reaktionsbedingungen vorliegen. Es können sowohl unpolare aprotische als auch polare aprotische Lösungsmittel eingesetzt werden.

Beispiele für bevorzugte unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methyl-cycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethyl-naphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Beispiele für geeignete polar-aprotische Lösungsmittel sind N,N-disubstituierte aliphatische Carbonsäureamide (insbesondere N,N-Di-C₁-C₄-alkyl-C₁-C₄-carbonsäureamide), stickstoffhaltige Heterocyclen und aprotische Ether (insbesondere cyclische Ether, Di-arylether und Di-C₁-C₆-alkylether von monomeren und oligomeren C₂-C₃-Alkylenglyko-len, die bis zu 6 Alkylenoxideinheiten enthalten können, vor allem Diethylenglykoldi-C₁-C₄-alkylether).

Als Beispiele für besonders geeignete Lösungsmittel seien im einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Tetrahydrofuran, Dioxan, Diphenylether, Diethylenglykoldi-methyl-, - diethyl-, -dipropyl-, -diisopropyl-, -di-n-butyl-, -di-sec.-butyl- und -di-tert.-butyl-ether, Diethylenglykolmethylethylether, Triethylenglykoldimethyl- und -diethylether und Triethylenglykolmethylethylether.

Die Lösungsmittelmenge beträgt in der Regel 10 bis 1000 ml, bevorzugt 20 bis 300 ml, pro g halogeniertem Aromat.

Als Übergangsmetallkatalysator eignen sich insbesondere Palladiumkomplexe, die wiederum in der Regel in Mengen von 1 bis 20 mol%, vor allem 2 bis 10 mol-%, bezogen auf den halogenierten Aromaten eingesetzt werden. Die zusätzliche Anwesenheit von freien Ligandmolekülen ist üblicherweise nicht erforderlich.

Beispiele für solche Katalysatoren sind Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)-ferrocen]palladium(II)chlorid, Bis(triethylphosphin)palladium(II)chlorid, Bis(tricyclo-hexylphosphin)palladium(II)acetat, (2,2'-Bipyridyl)palladium(II)chlorid, Bis(triphenyl-phosphin)palladium(II)chlorid, Tris(dibenzylidenaceton)dipalladium(0), 1,5-Cycloocta-dienpalladium(II)chlorid, Bis(acetonitril)palladium(II)chlorid und Bis(benzonitril)palla-dium(II)chlorid, Palladium(II)acetat, wobei [1,1'-Bis(diphenylphosphino)ferrocen]palladium(II)chlorid, Tetrakis(triphenylphosphin)palladium(0) und Palladium(II)acetat bevorzugt sind.

In der Regel empfiehlt sich die gleichzeitige Anwesenheit freier Ligandmoleküle, z.B. von Tri(tert.-butyl)phosphin, Tri(i-butyl)phosphin, Triphenylphosphin und Tris(o-tolyl)phosphin und 2-Dicyclohexylphosphino-2,6-dimethoxybiphenyl. Übliche Mengen liegen bei 80 bis 500 mol%, bevorzugt 100 bis 300 mol-%, bezogen auf den Übergangsmetallkatalysator. Als Base kommen vorzugsweise die Alkalimetallsalze, insbesondere die Natrium- und vor allem die Kaliumsalze, schwacher organischer und anorganischer Säuren, wie Natriumacetat, Kaliumacetat, Natriumcarbonat, Natriumhydrogencarbonat, Kaliumcarbonat und Kaliumhydrogencarbonat, Phosphat, Fluoride wie Kaliumfluorid, zum Einsatz. Bevorzugte Basen sind die Acetate, vor allem Kaliumacetat.

Im Allgemeinen werden 1 bis 5 mol, bevorzugt 2 bis 4, mol, Base pro mol halogeniertem Aromaten verwendet.

Die Reaktionstemperatur liegt üblicherweise bei 20 bis 180°C, vor allem bei 60 bis 120°C.

Die Reaktionszeit beträgt in der Regel 0,5 bis 30 h, insbesondere 1 bis 20 h.

Vefahrenstechnisch geht man bei der Herstellung der Boronsäurederivate zweckmäßigerweise wie folgt vor:

Man legt den halogenierten Aromaten und Lösungsmittel vor, gibt das Diboran der allgemeinen Formel (IV), den Übergangsmetallkatalysator und die Base nacheinander zu und erhitzt die Mischung 0,5 bis 30 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Die Suzuki-Reaktion des so hergestellten Boronsäurederivates mit dem entsprechenden halogenierten Aromaten kann grundsätzlich zur Herstellung des Produktes in Schritt (a) des erfindungsgemäßen Verfahrens unter analogen Bedingungen verwendet werden, wobei an Stelle des Diborans das entsprechende Boronsäurederivat mit dem entsprechenden halogenierten Aromaten umgesetzt wird.

Vorzugsweise erfolgt jedoch die Umsetzung des Boronsäurederivates mit dem halogenierten Aromaten in Gegenwart eines organischen Lösungsmittels, gewünschtenfalls im Gemisch mit Wasser, sowie eines Übergangsmetallkatalysators und einer Base, wobei das Molverhältnis von Boronsäurederivat zu halogeniertem Aromaten dabei in der Regel 0,8 : 1 bis 3 : 1, vorzugsweise 0,9 : 1 bis 2 : 1 beträgt.

Als Lösungsmittel eignen sich alle Lösungsmittel, in denen sich die Edukte bei Reaktionstemperatur vollständig und die verwendeten Katalysatoren und Basen zumindest partiell lösen, so dass weitgehend homogene Reaktionsbedingungen vorliegen.

Beispielsweise geeignet sind Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methyl-cycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethyl-naphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Die Lösungsmittelmenge liegt üblicherweise bei 10 bis 1000 ml, vorzugsweise bei 20 bis 100 ml, je g Boronsäurederivat.

Vorzugsweise setzt man Wasser als zusätzliches Lösungsmittel ein. In diesem Fall werden in der Regel 10 bis 1000 ml, insbesondere 250 bis 500 ml, Wasser je I organisches Lösungsmittel verwendet.

Als Übergangsmetallkatalysatoren werden ebenfalls vorzugsweise Palladiumkomplexe eingesetzt. Die Einsatzmenge Katalysator beträgt üblicherweise 1 bis 20 mol%, insbesondere 1,5 bis 5 mol-%, bezogen auf das Boronsäurederivat.

In der Regel empfiehlt sich die gleichzeitige Anwesenheit freier Ligandmoleküle, z.B. von Tri(tert.-butyl)phosphin, Tri(i-butyl)phosphin, Triphenylphosphin und Tris(o-tolyl)phosphin und 2-Dicyclohexylphosphino-2,6-dimethoxybiphenyl. Übliche Mengen liegen bei 80 bis 500 mol%, bevorzugt 100 bis 300 mol-%, bezogen auf den Übergangsmetallkatalysator.

Als Base sind Alkalimetallsalze schwacher Säuren bevorzugt, wobei die Carbonate, wie Natriumcarbonat und vor allem Kaliumcarbonat besonders bevorzugt sind. Auch Phosphate, wie Natrium- oder Kaliumphosphat, sind hierbei bevorzugt. In der Regel liegt die Basenmenge bei 0,1 bis 10 mol, insbesondere bei 0,2 bis 5 mol, je mol Boronsäurederivat. Die Reaktionstemperatur beträgt im Allgemeinen 20 bis 180°C, bevorzugt 60 bis 120°C. Wird Wasser eingesetzt, so empfiehlt es sich, die Umsetzung nicht bei Temperaturen über 100°C vorzunehmen, da ansonsten unter Druck gearbeitet werden müsste.

Die Reaktion ist üblicherweise in 0,5 bis 48 h, insbesondere in 5 bis 20 h, beendet.

Verfahrenstechnisch geht man zweckmäßigerweise wie folgt vor:

Man legt das Boronsäurederivat und den halogenierten Aromaten sowie Lösungsmittel vor, gibt Übergangsmetallkatalysator und die vorzugsweise in Wasser oder einem Wasser/Alkohol-Gemisch gelöste Base zu und erhitzt die Mischung 0,5 bis 48 h unter Schutzgas auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Die Reinheit des so hergestellten Produktes aus Schritt (a) des erfindungsgemäßen Verfahrens zur Herstellung der Heptarylen- und Octarytentetracarbonsäurediimide reicht im allgemeinen für die weitere Umsetzung in Schritt (b) aus. Gegebenenfalls kann das Rohprodukt durch Waschen mit Wasser und gewünschtenfalls einem geeigneten organischen Lösungsmittel, insbesondere einem chlorierten aliphatischen oder aromatischen Kohlenwasserstoff, oder durch Säulenchromatographie an Kieselgel mit einem Gemisch von Methylenchlorid und Hexan oder Pentan oder mit Toluol als Eluens weiter aufgereinigt werden.

Die Ausbeute in Schritt a) des erfindungsgemäßen Verfahrens liegt üblicherweise bei 50 bis 90%.

Neben der oben beschriebenen Suzuki-Reaktion, die ein entsprechendes Boronsäurederivat erfordert, kann auch eine direkte Kupplung, insbesondere bei Homokupplungen, von Halogeniden erfolgen.

Hierbei kann die Umsetzung der entsprechend halogenierten Aromaten der Formel (II) und (III) in Gegenwart eines Diborans der allgemeinen Formel (IV) erfolgen. Dabei läuft schließlich ebenfalls eine Suzuki-Reaktion ab, wobei jedoch das entsprechende Boronsäurederivat nur in situ erzeugt wird.

Die ·Kupplung kann dabei beispielsweise in Gegenwart von 30 bis 70 mol-%, bezogen auf das den halogenierten Aromaten, eines Diborans der allgemeinen Formel (IV), eines Übergangsmetallkatalysators, einer Base und eines aprotischen Lösungsmittels gemäß einer Suzuki-Kupplungsreaktion erfolgen, wobei das in situ gebildete Boronsäurederivat nicht zwischenisoliert, sondern direkt mit dem verbleibenden halogenierten Aromaten umgesetzt wird.

Bei dieser Verfahrensvariante wird analog wie oben vorgegangen, wobei jedoch beispielsweise nur 30 bis 70 mol-% Diboran der allgemeinen Formel (IV), bezogen auf den halogenierten Aromaten eingesetzt werden.

In der Regel werden 1 bis 20 mol-%, bevorzugt 5 bis 10 mol-%, Übergangsmetallkatalysator und 1 bis 5 mol, vorzugsweise 2 bis 3 mol, Base je mol halogeniertem Aromaten verwendet. Das aprotische organische Lösungsmittel kommt üblicherweise in Mengen von 10 bis 100 ml, insbesondere 20 bis 50 ml, je g halogeniertem Aromaten zum Einsatz.

Die Reaktionstemperatur liegt im Allgemeinen bei 20 bis 100°C, vorzugsweise bei 60 bis 80°C, und die Reaktionsdauer bei 12 bis 72 h, bevorzugt 24 bis 48 h.

Verfahrenstechnisch geht man zweckmäßigerweise wie folgt vor:

Man legt den halogenierten Aromaten und Lösungsmittel vor, gibt dass Diboran der allgemeinen Formel (IV), den Übergangsmetallkatalysator und die Base nacheinander zu und erhitzt die Mischung 12 bis 72 h auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Auch hier reicht die Reinheit des erhaltenen Produktes in der Regel für die nachfolgende Cyclodehydrierung in Schritt (b) des erfindungsgemäßen Verfahrens aus. Eine weitere Aufreinigung ist beispielsweise durch Säulenchromatographie möglich.

Die Ausbeute liegt üblicherweise bei 80 bis 95%.

Darüber hinaus besteht die Möglichkeit eine direkte Kupplung der halogenierten Aromaten durchzuführen, ohne dass ein Diboran eingesetzt wird.

Diese Kupplung kann dabei beispielsweise in Gegenwart eines organischen Übergangmetallkomplexes als Katalysator, freier Ligandmoleküle und eines aprotischen Lösungsmittels im Sinne einer Homo-Kupplung erfolgen.

Geeignete inerte Verdünnungsmittel sind zum Beispiel aliphatische Carbonsäureamide, wie N, N-Dimethylformamid und N, N-Dimethylacedamid, aliphatische und cycloaliphatische Ether, wie 1, 2-Dimethoxyethan, und Aromaten, wie Benzol, Toluol und Xylol, wobei N, N-Dimethylformamid und N, N-Dimethylacedamid bevorzugt sind.

Die Verdünnungsmittelmenge beträgt in der Regel 20 bis 100 g, vorzugsweise 25 bis 45 g je Gramm Halogenverbindung.

Bei den als Katalysator dienenden organischen Übergangsmetallkomplexen kommen neben den bekannten Palladiumkomplexen, wie Tetrakis(triphenylphosphin)palladium(0) insbesondere Nickelkomplexe in Betracht, zum Beispiel Bis(triphenylphosphin)nickel(II)chlorid, Tetrakis(triphenylphosphin)nickel(0), [1,2-Bis(diphenylphosphino)ethan]nickel(II)chlorid und bevorzugt Bis(1,5-cyclooktadien)nickel(0). Man kann die Katalysatoren auch durch die Zugabe von Übergangsmetallsalzen oder -verbindungen, freien Liganden wie Cyclooctadien, Bipyridyl, Triphenylphosphin, Trifluorphosphin, η-, δ- und π-gebundenen Olefinen, Cycloolefinen, Aromaten und Antiaromaten, Carbonylen, Wasserstoff und Halogen sowie auch deren Mischungen und erforderlichenfalls Oxidations- und Reduktionsmitteln erzeugen.

Im Allgemeinen werden 40 bis 150 mol%, vorzugsweise 50 bis 100 mol-%, organischer Übergangsmetallkomplex bezogen auf die eingesetzte Halogenverbindung eingesetzt.

In der Regel empfiehlt sich stets die gleichzeitige Anwesenheit von freien Ligandmolekülen, wie insbesondere Mischungen von Cyclooctadien und Bipyridyl im molaren Verhältnis von 1 : 1 bis 8 : 1. Hierbei sind Mengen von üblicherweise 80 bis 900 mol-%, bevorzugt 80 bis 200 mol%, bevorzugt bezogen auf die Halogenverbindung geeignet.

Die Kupplungstemperatur beträgt im Allgemeinen 40 bis 80°C, vorzugsweise 60 bis 70°C.

Die Reaktionszeit liegt in der Regel bei 24 bis 48 h, insbesondere bei 36 bis 48 h.

Verfahrenstechnisch geht man bei dieser direkten Kupplung zweckmäßigerweise so vor, dass man die Halogenverbindung, den metallorganischen Katalysator sowie freie Ligandmoleküle im inerten Verdünnungsmittel vorlegt und, ggf. unter Schutzgas 24 bis 48 h auf die gewünschte Reaktionstemperatur erhitzt. Nach Abkühlen trägt man das Reaktionsgemisch in Wasser, das ggf. Methanol enthalten kann, ein, gibt verdünnte anorganische Säure, z. B. verdünnte Salzsäure, zu und filtriert den gebildeten Niederschlag ab, wäscht mit Wasser und trocknet im Vakuum.

Die Reinheit des so hergestellten erfindungsgemäßen Produktes reicht im Allgemeinen für die nachfolgende Cyclodehydrierung in Schritt (b) des erfindungsgemäßen Verfahrens aus. Gegebenenfalls kann das Produkt noch zusätzlich durch eine Säulenchromatographie an Kieselgel mit einem Gemisch von Methylenchlorid und Hexan oder Pentan als Elluenz weiter aufgereinigt werden.

Die Ausbeute liegt im Allgemeinen bei 70 bis 90%.

In Schritt (b) des erfindungsgemäßen Verfahrens findet die Cylclodehydrierung des in Schritt (a) erhaltenen Umsetzungsprodukts statt. Die Cyclodehydrierung kann in einem Hydroxy- und Aminofunktionen aufweisenden und eine im Wesentlichen ungelöste Base enthaltenden organischen Reaktionsmedium oder in Gegenwart eines basenstabilen, hochsiedenden, organischen Lösemittels sowie einer alkali- oder erdalkalimetallhaltigen Base und einer stickstoffhaltigen Hilfsbase vorgenommen werden.

Bevorzugt ist die erstgenannte Verfahrensvariante. Hierbei sind als organisches Reaktionsmedium vor allem Aminoalkohole geeignet, die 2 bis 20, vorzugsweise 2 bis 10 Kohlenstoffatome aufweisen. Die Kohlenstoffkette dieser Alkohole kann durch Sauerstoffatome in Etherfunktion unterbrochen sein. Beispiele für besonders geeignete Lösungsmittel sind Ethanolamin, Triethanolamin und Diethanolamin, wobei Ethanolamin bevorzugt ist. Es ist auch möglich, Mischungen von Alkoholen und Aminen zu verwenden, die jeweils einen Siedepunkt von mindestens 70°C haben und bei der Reaktionstemperatur flüssig sind.

Üblicherweise werden 1,5 bis 150 ml, bevorzugt 5 bis 50 ml, Reaktionsmedium je Gramm Ausgangsverbindung eingesetzt.

Als im Reaktionsmedium wesentlich unlösliche Base eignen sich die Alkalimetallsalze, insbesondere die Natriumsalze und v.a. die Kaliumsalze, schwacher organischer und bevorzugt schwacher anorganischer Säuren, wie Formiate, Acetate, Propionate, Hydrogencarbonate und besonders bevorzugt Karbonate, insbesondere Natriumkarbonat und v.a. Kaliumkarbonat.

In der Regel beträgt die Basenmenge 1 bis 10 mol, bevorzugt 2 bis 5 mol, je Mol Ausgangsverbindung.

Die Reaktionstemperatur liegt im Allgemeinen bei 40 bis 200°C, insbesondere bei 80 bis 160°C.

Die Reaktionszeit beträgt üblicherweise 0,5 bis 24 h, vorzugsweise 1 bis 12 h.

Verfahrenstechnisch geht man zweckmäßigerweise so vor, dass man eine Mischung Ausgangsverbindung, Lösungsmittel und Base 0,5 bis 24 h unter Schutzgas bei der gewünschten Reaktionstemperatur rührt und das gebildete erfindungsgemäße Produkt der Formel (I) nach Abkühlen auf Raumtemperatur durch Zugabe eines Alkohols, wie -Ethanol, oder von Wasser aus dem Reaktionsgemisch ausfällt, abfiltriert und mit Wasser wäscht.

Die Reinigung der erfindungsgemäßen Diimide kann dadurch erfolgen, dass Katalysatorrückstände durch eine schnelle Filtration über Kieselgel unterwaschen mit einem halogenierten aliphatischen Kohlenwasserstoff, wie Methylenchlorid, entfernt werden. Rückstände nicht umgesetzter Edukte können durch Säulenchromatographie an Kieselgel mit Methylenchlorid als Eluenz oder durch wiederholtes Waschen mit Hexan oder Pentan entfernt werden.

Die Ausbeute liegt im Allgemeinen bei 90 bis 100%.

Als Produkt nach dem erfindungsgemäßen Verfahren zur Herstellung von Heptarylen- und Octarylentetracarbonsäuredimiden werden Diimide der allgemeinen Formel (I) 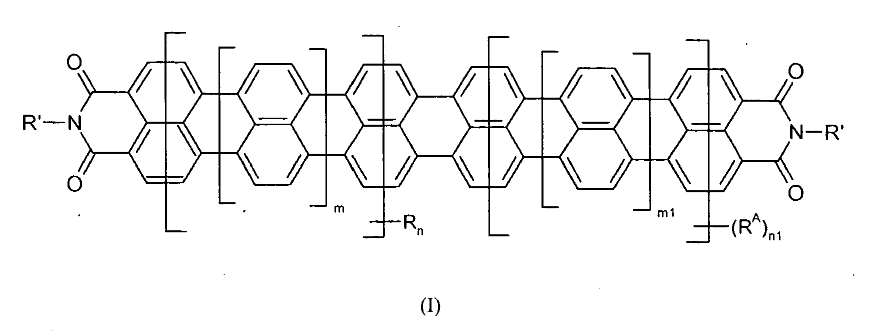 oder deren Mischungen erhalten.

### Hierbei haben die Variablen R, R^{A}, R' folgende Bedeutung:

Jedes R, R^{A} unabhängig voneinander sind gleiche oder verschiedene der folgenden Reste:
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
   (i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder - SO₂- unterbrochen sein kann und das ein- oder mehrfach substituiert sein kann durch: C₁-C₁₂-Alkoxy, C₁-C₆-Alkylthio, -C≡CR¹,
      -CR¹=CR¹₂, Hydroxy, Mercapto, Halogen, Cyano, Nitro, -NR²R³,
      -NR²COR³, -CONR²R³, -SO₂NR²R³, -COOR², -SO₃R², -PR²R³ ,-POR²R³, Aryl und/oder gesättigtes oder ungesättigtes C₄-C₇-Cycloalkyl, dessen Kohlenstoffgerüst durch eine oder mehrere Gruppierungen -O-, -S-,
      -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
   (ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder - SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder - SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², - SO₃R², -PR²R³ und/oder -POR²R³;
   (iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, - CONR²R³, -SO₂NR²R³, -COOR², -SO₃R², -PR²R³ und/oder -POR²R³ substituiert sein kann;
   (iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
   (v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, - COOR², -SO₃R², -PR²R³ und/oder -POR²R³;
jedes R' ist jeweils unabhängig Wasserstoff;
   C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder - SO₂- unterbrochen sein kann und das ein- oder mehrfach durch die als Substituenten für die Reste R genannten Reste (ii), (iii), (iv) und/oder (v) substituiert sein kann;
   C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem durch die als Substituenten für die Reste R genannten Reste (i), (ii), (iii), (iv) und/oder (v) substituiert sein kann; oder
   Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem substituiert sein kann durch die als Substituenten für die Reste R genannten Reste (i), (ii), (iii), (iv), (v), Aryl- und/oder Hetarylazo, das jeweils durch C₁-C₁₀-Alkyl, C₁-C₆-Alkoxy und/oder Cyano substituiert sein kann.
Hierbei haben die Variablen R", R¹ bis R² die folgende Bedeutung:
   Jedes R" ist unabhängig voneinander Wasserstoff, C₁-C₃₀-Alkyl, C₅-C₈-Cycloalkyl, Aryl oder Hetaryl oder miteinander verbunden unter Ausbildung eines die beiden Sauerstoffatome sowie das Boratom enthaltenden 5- bis 7-gliedrigen Rings, an den ungesättigte oder gesättigte Ringe anneliert sein können und der an den Kohlenstoffatomen durch bis zu 4 C₁-C₃₀-Alkyl-, C₅-C₈-Cycloalkyl-, Aryl- oder Hetarylgruppen substituiert sein kann;
   R¹ ist Wasserstoff oder C₁-C₁₈-Alkyl, wobei die Reste R¹ gleich oder verschieden sein können, wenn sie mehrfach auftreten;
   R², R³ sind unabhängig voneinander Wasserstoff;
      C₁-C₁₈-Alkyl, dessen Kohlenstoffkette durch eine oder mehrere Gruppie-rungen -O-, -S-, - CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
      Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann.

Als Beispiele für die in den Formeln genannten Reste R, R^{A}, R', R", R¹ bis R³ sowie deren Substituenten seien im Einzelnen genannt:

Beispiele für Alkyle sind Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen); Die als Indizes angegebenen Zahlen nach dem Symbol "C" beziehen sich auf die Mindest- und Höchstanzahl der C-Atome der Alkyle.

Beispiele für durch Sauerstoff unterbrochene Alkyle sind 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxy-propyl, 2- und 4-Methoxybutyl, 2- und 4-Ethoxybutyl, 2- und 4-Propoxybutyl, 3,6-Di-oxaheptyl, 3,6-Dioxaoctyl, 4,8-Dioxanonyl, 3,7-Dioxaoctyl, 3,7-Dioxanonyl, 4,7-Dioxa-octyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxa-tetradecyl;

Beispiele für durch Schwefel unterbrochene Alkyle sind 2-Methylthioethyl, 2-Ethylthioethyl, 2-Propylthioethyl, 2-Isopropylthioethyl, 2-Butylthio-ethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Pro-pylthiobutyl, 3,6-Dithiaheptyl, 3,6-Dithiaoctyl, 4,8-Dithianonyl, 3,7-Dithiaoctyl, 3,7-Di-thianonyl, 2- und 4-Butylthiobutyl, 4,8-Dithiadecyl, 3,6,9-Trithiadecyl, 3,6,9-Trithia-undecyl, 3,6,9-Trithiadodecyl, 3,6,9,12-Tetrathiatridecyl und 3,6,9,12-Tetrathiatetra-decyl;

Beispiele für durch Aminogruppen unterbrochene Alkyle sind 2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Di-azaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;

Weitere Beispiele für Alkylgruppen, die unterbrochen und/oder Substituenten aufweisen, sind
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulfoxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonyl-ethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonyl-butyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Beispiele für Alkyloxy sind Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Beispiele für Alkylthio sind Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Beispiele für Reste mit Dreifachbindung sind Ethinyl, 1- und 2-Propinyl, 1-, 2- und 3-Butinyl, 1-, 2-, 3- und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl;
Beispiele für Reste mit Doppelbindung sind Ethenyl, 1- und 2-Propenyl, 1-, 2- und 3-Butenyl, 1-, 2-, 3- und 4-Pentenyl, 1-, 2-, 3-, 4- und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6- , 7-, 8-, 9-, 10-, 11-, 12-, 13-, 14-, 15-, 16- und 17-Octadecenyl;

Beispiele für weitere Reste sind
Methylamino, Ethylamino, Propylamino, Isopryplamino, Butylamino, Isobutylamino, Pentylamino, Hexylamino, Dimethylamino, Methylethylamino, Diethylamino, Dipropyl-amino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylamino-carbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butyl-phenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Halogene sind Chlor, Brom und lod;
Aryl- bzw. Hetarylazo sind beispielsweise Phenylazo, 2-Napthylazo, 2-Pyridylazo und 2-Pyrimidylazo;

Gegebenenfalls substituierte Cycloalkyle sind beispielsweise Cyclopropyl, Cyclobutyl, Cyclopentyl, 2- und 3-Methylcyclopentyl, 2- und 3-Ethylcyclo-pentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methyl-cycloheptyl, 2-, 3- und 4-Ethylcycloheptyl, 3- und 4-Propylcycloheptyl, 3- und 4-Iso-propylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl. Die als Indizes angegebenen Zahlen nach dem Symbol "C" beziehen sich auf die Mindest- und Höchstanzahl der C-Atome der Cycloalkyle.

Beispiele für gegebenenfalls unterbrochene Cycloalkyle sind
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Gegebenenfalls kondensierte und/oder substituierte und/oder unterbrochene Aryl- und Hetarylgruppen sollten mindestens 3 bis 14 Ringatome aufweisen, vorzugsweise 5 bis 10 Ringatome, und sind beispielsweise
Phenyl, 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothia-zolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methylisoindolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroiso-chinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethyl-chinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butyl-phenyl; 2-, 3- und 4-Methoxyphenyl, 2,4-, 3,5- und 2,6-Dimethoxyphenyl, 2,4,6-Tri-methoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxy-phenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamido-phenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylamino-phenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)amino-phenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;

Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Vorzugsweise handelt es sich bei den erfindungsgemäßen Diimiden der allgemeinen Formeln (I) oder Mischungen davon um solche, bei denen R und R^{A} die gleiche Bedeutung besitzen.

Ebenfalls bevorzugt handelt es sich bei den erfindungsgemäßen Diimiden der allgemeinen Formeln (I) oder Mischungen davon um solche, bei denen R, R^{A} unabhängig voneinander Aryloxy oder Arylthio sind, wobei das gesamte Ringsystem ein- oder mehrfach durch die Reste (i), (ii), (iii), (iv) und/oder (v) wie oben angegeben, substituiert sein kann. Insbesondere bevorzugt ist, wenn R, R^{A} unabhängig voneinander ein- oder mehrfach durch einen Rest (i) substituiert sein kann.

Ebenfalls bevorzugt handelt es sich bei den erfindungsgemäßen Diimiden der allgemeinen Formeln (I) oder Mischungen davon um solche, bei denen R, R^{A} unabhängig voneinander 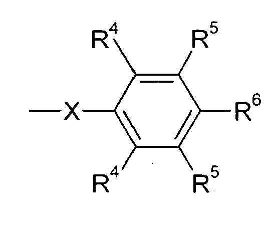 sind,
wobei
X O oder S ist und
R⁴, R⁵, R⁶ unabhängig voneinander Wasserstoff oder die Reste (i), (ii), (iii), (iv) und/oder (v) wie oben angegeben mit der Maßgabe sein können, dass zumindest einer der Reste R⁴, R⁶ kein Wasserstoff ist. Insbesondere bevorzugt ist, dass wenn R⁴ C₁-C₃₀ Alkyl oder C₃-C₈ Cycloalkyl ist, in der 1-Position kein ternäres Kohlenstoffatom auftritt.

Weiterhin bevorzugt ist, dass beide R⁴ kein Wasserstoff und R⁵, R⁶ Wasserstoff sind oder dass R⁶ kein Wasserstoff ist und R⁴, R⁵ Wasserstoff sind.

Ebenfalls bevorzugt handelt es sich bei den erfindungsgemäßen Diimiden der allgemeinen Formeln (I) oder Mischungen davon um solche, bei denen jedes R' unabhängig voneinander C₁-C₃₀ Alkyl oder Aryl ist, wobei das gesamte Ringsystem ein- oder mehrfach durch die Reste (i), (ii), (iii), (iv) und/oder (v) wie oben angegeben, substituiert sein kann. Insbesondere bevorzugt ist R' ein- oder mehrfach durch einen Rest (i) substituiert. Ebenfalls bevorzugt sind alle R' gleich.

Die erfindungsgemäßen Diimide der allgemeinen Formel (I) zeigen starke Absorption im Infrarotbereich bei Wellenlängen von 750 bis 1300 nm. Ihre Funktionalisierung kann gezielt gewählt werden, so dass sie direkt an den gewünschten Verwendungszweck angepasst werden können.

Sie eignen sich für eine Vielzahl von Anwendungen, wie die Einfärbung von hochmolekularen organischen und anorganischen Materialien, z.B. von Lacken, Druckfarben und Kunststoffen, zur Herstellung im nahinfraroten Bereich des elektromagnetischen Spek-trums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-laserstrahlen-absorbierende Materialien beim Schweißbehandeln von Kunststoffteilen, als Halbleiter in der organischen Elektronik, als Emitter in Elektro- und Chemilumineszenzanwendungen sowie als Aktivkomponenten in der Photovoltaik.

Ein weiterer Gegenstand der vorliegenden Erfindung ist ein Verfahren zur Herstellung einer Quaterrylenverbindung der allgemeinen Formel (II) 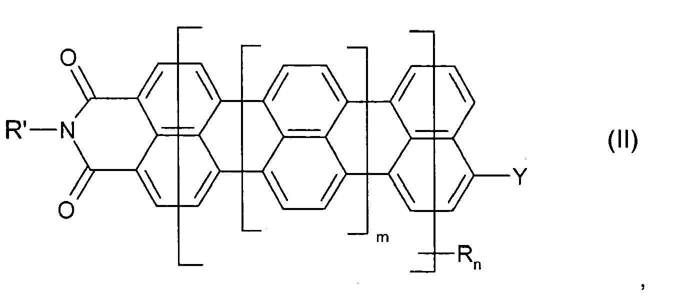 wobei Y gleich Halogen und R', R, n, m die oben angegebene Bedeutung besitzen, die Schritte enthaltend
(a) Kupplung mindestens einer Perylenverbindung der Formel (II) 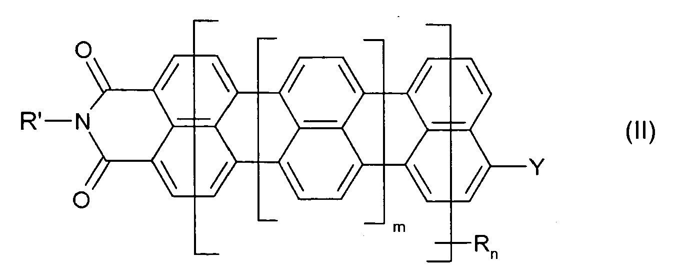 mit mindestens einer Verbindung der Formel (IIIa) und/oder (IIIa') 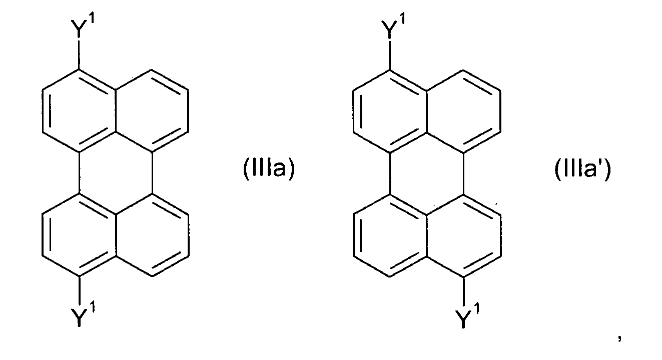 wobei
   ein Rest von Y, Y¹ Halogen und der andere B(OR")₂ ist, m = 0 ist und R, R', n, die oben angegebene Bedeutung besitzen, zu einer Verbindung der allgemeinen Formel (IIIb)/(IIIb') 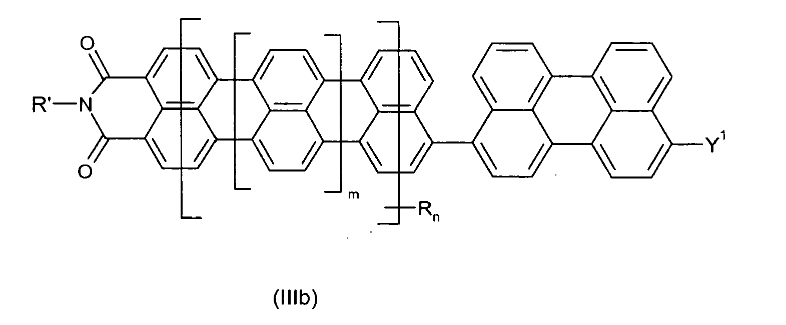 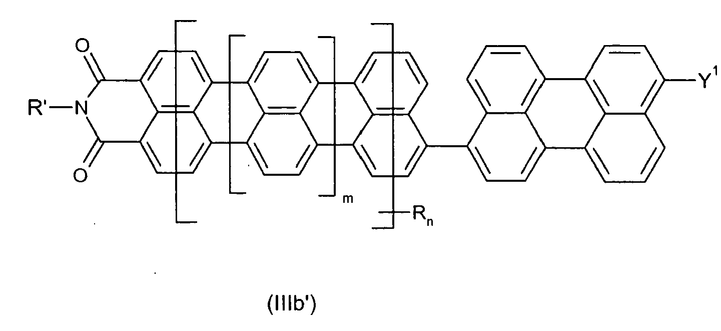 und
(b) Cyclodehydrierung der in Schritt (a) erhaltenen Verbindung der allgemeinen Formel (IIIb)/(IIIb') zu einer Rylenverbindung der allgemeinen Formel (II), wobei Y¹ in Formel (IIIb)/(IIIb') die Bedeutung von Y in Formel (II) hat.

Solche Rylenverbindungen der Formel (II) eignen sich, wie oben erwähnt, als Ausgangsverbindungen zur Herstellung der Heptarylen- und Octarylentetracarbonsäurediimide und können mit Hilfe von Verbindungen der Formel (IIIb)/(IIIb') erhalten werden, wobei die Verbindungen der Formel (IIIb)/(IIIb') bislang nicht bekannt waren.

Ein weiterer Gegenstand der vorliegenden Erfindung ist daher eine Verbindung der allgemeinen Formel (IIIb) oder (IIIb') 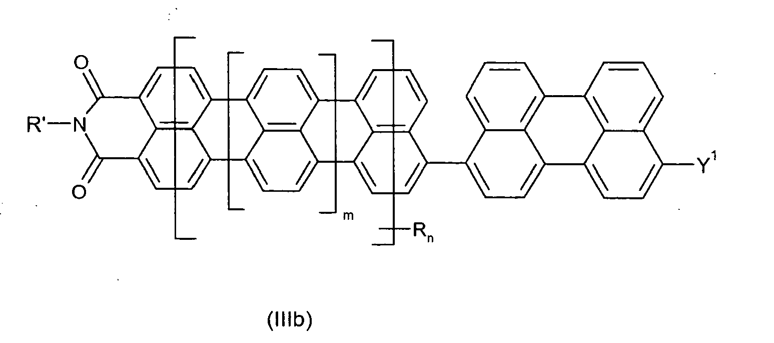 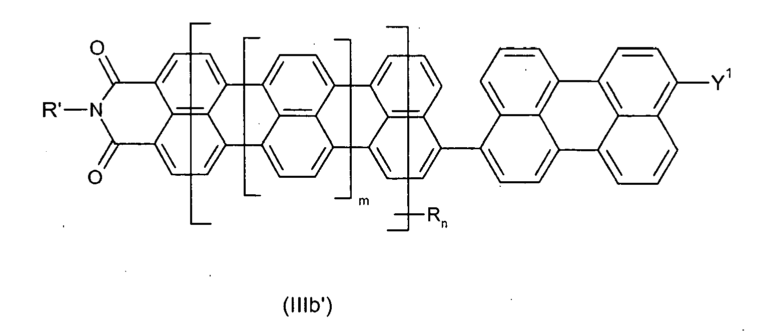 oder Mischungen davon, wobei Y¹ Halogen oder B(OR")₂ ist, m = 0 ist und R, R', n, die oben angegebene Bedeutung besitzen. Vorzugsweise ist hierbei Y¹ Halogen, insbesondere Brom, so dass es sich bei der Verbindung der Formel (IIIa) um 3,9(10)-Dibromperylen handelt.

Bei den Reaktionen der Schritte (a) und (b) handelt es sich wie weiter oben ausgeführt um eine Suzuki-Reaktion und eine Cyclodehydrierung, so dass das oben ausgeführte grundsätzlich analog gilt.

Vorzugsweise wird dabei in Schritt (a) die Verbindung der Formel (IIIa) im Überschuss eingesetzt. Vorzugsweise beträgt der Überschuss mindestens das Siebenfache. In Schritt (b) erfolgt die Umsetzung bevorzugt in Gegenwart eines Metallsalzes, wie beispielsweise Eisen(III)-chlorid.

### Beispiele

### Beispiel 1

### Herstellung von N-(2,6-Diisopropylphenyl-1,6-bis[4-(1,1,3,3-tetramethylbutyl)-phenoxy]-9-(7-[1 (9)-bromo]- perylen)perylen-3,4-dicarbonsäureimid

Zu einer unter Argon gerührten Mischung von 0,51g (0,5 mmol) N-(2,6-Diisopropylphenyl-4-(4,4,5,5-tetramethyl-1,3,2-dioxaboran-2-yl)-perylen-3,4-dicarbonsäureimid und 1,43 g (3,5 mmol) 3,9(10)-dibromoperylen in 40 mL Toluol und 2mL Ethanol wurden zuerst eine Lösung von 0,5 mL Kaliumcarbonat in Wasser (2M) und dann 0,03 g (0,03 mmol) Pd(PPh₃)₄ zugegeben. Die Mischung wurde unter Argon auf 70°C erhitzt und 6 h bei dieser Temperatur gerührt. Nach Abkühlen auf Raumtemperatur wurde die organische Phase abgetrennt und das Lösungsmittel im Vakuum abgezogen. Das Rohprodukt wurde einer Säulenchromatographie an Kieselgel mit Toluol als Eluens unterzogen.

Es wurden 0,42 g Produkt in Form eines roten Feststoffs erhalten, was einer Ausbeute von 69% entspricht.

Analytische Daten:
Rf = 0,72 (toluene);
MS (FD): m/z (rel. Int.) = 1220,6 (100%) [M+];
¹H-NMR (250 MHz, CD2Cl2, 25 °C): δ = 9,48 (d, 1H, PMI H-7), 9,35 (d, 1H, PMI H-12), 8,20-8,40 (m, 2H, Ar-H), 8,26 (s, 1H, PMI H-5), 8,24 (s, 1H, PMI H-2), 8,09 (t, 2H, Ar-H), 7,80 (d, 1H, Ar-H), 7,64 (dd, 3H, Ar-H), 7,51-7,54 (m, 2H, Ar-H), 7,44 (d, 4H, phenoxy m-H), 7,32 (d, 4H, phenoxy o-H), 7,06-7,35 (m, 6H, Ar-H), 2,74 (sep, 2H, CH i-propyl), 1,74 (s, 4H), 1,38 (s, 12H), 1,12 (d, 12H), 0,73 (s, 9H), 0,72 (s, 9H);
¹³C-NMR (62,9 MHz, CD₂Cl₂, 25 °C): 163,66, 154,20, 153,74, 146,45, 131,24, 130,09, 129,24, 128,46, 128,41, 127,31, 127,08, 124,40, 118,79, 118,63, 57, 41, 38,64, 32,60, 31,85, 31,67, 29,43, 24,09.

### Beispiel 2

### Herstellung von N-(2,6-Diisopropylphenyl-1,6 -bis[4-(1,1,3,3-tetramethylbutyl)-phenoxy]-13-bromo-quaterrylen-3,4-dicarbonsäureimid

Zu einer Lösung von 0,2 g (0,16 mmol) Produkt aus Beispiel 1 in 10 ml Methylenchlorid wurde eine Lösung von 0,41 g (2,56 mmol) Eisen(III)chlorid in 2 ml wasserfrei Nitromethan unter Argon zugetropft. Nach einer Reaktionszeit von 24 h bei Raumtemperatur wurde das Lösungsmittel abgezogen, die Reaktionsmischung mit wäßriger Salzsäure versetzt, filtriert und mit Methanol gewaschen. Das Rohprodukt wurde einer Säulenchromatographie an Kieselgel mit Dichlormethan als Eluens unterzogen.

Es wurden 0,12 g Produkt in Form eines grünen Feststoffs erhalten, was einer Ausbeute von 60% entspricht.

Analytische Daten:
Rf = 0,58 (toluene);
MS (FD): m/z (rel.int.) 1218,8 (100%), M+, 609,9 (40%), M/2;
UV-Vis (CH₂Cl₂): λₘₐₓ (ε): 732 (177100), 668 (78000), 359 (17200), 260 (122000)

### Beispiel 3

### Herstellung von N-(2,6-Diisopropylphenyl-1,6,11,15-tetra[4-(1,1,3,3-tetramethylbutyl)-phenoxy]-13-(11-[N-(2,6-diisopropylphenyl)]-1,6-bis[2,6-diisopropylphenoxy]terrylen-3,4-dicarbonsäureimid)quaterrylen-3,4-dicarbonsäureimid.

Zu einer unter N₂ gerührten Mischung von 0,057g (0,04 mmol) N-(2,6-Diisopropylphenyl-1,6,9,14-tetra[2,6-diisopropylphenoxy] -11-(4,4,5,5-tetramethyl-1,3,2-dioxaboran-2-yl)- terrylen-3,4-dicarbonsäureimid und 0,063 g (0,04 mmol) N-(2,6-Diisopropylphenyl-1,6,11,15-tetra[4-(1,1,3,3-tetramethylbutyl)-phenoxy]-13-chloro-quaterrylen-3,4-dicarbonsäureimid in 15 ml Toluol wurden zuerst eine Lösung von 0,038 g (0,28 mmol) Kaliumcarbonat in 4 ml Wasser und 0,4 mL Ethanol und dann 0,001 g (0,004 mmol) Palladium(II)-acetat und 0,008 g (0,003 mmol) 2-Dicyclohexylphosphino-2,6-dimethoxybiphenyl zugegeben. Die Mischung wurde unter N₂ auf 85°C erhitzt und 14 h bei dieser Temperatur gerührt. Nach Abkühlen auf Raumtemperatur wurde die organische Phase abgetrennt und das Lösungsmittel im Vakuum abgezogen. Das Rohprodukt wurde einer Säulenchromatographie an Kieselgel mit Toluol als Eluens unterzogen.

Es wurden 60 mg Produkt in Form eines schwarzgrünen Feststoffs erhalten, was einer Ausbeute von 52% entspricht.

Analytische Daten:
Rf = 0,2 (Toluol);
MS (Maldi): m/z (rel. Int.) = 2853,5(100%) [M+].

### Beispiel 4

### Herstellung von N-(2,6-Diisopropylphenyl-1,6-bis[4-(1,1,3,3-tetramethylbutyl)-phenoxy]-13-(13-[N-(2,6-diisopropylphenyl)]-1,6-bis[4-(1,1,3,3-tetramethylbutyl)-phenoxy]quaterrylen-3,4-dicarbonsäureimid)quaterrylen-3,4-dicarbonsäureimid

Zu einer gerührten Mischung von 40 mg Ni(cod)2 und 0,1 mL Cyclooctadiene und 24 mg 2,2'-bispyridyl in 15 ml wasserfrei DMF wurden eine Lösung von 0,017 g (0,1 mmol) N-(2,6-Diisopropylphenyl-1,6-bis[4-(1,1,3,3-tetramethylbutyl)-phenoxy]-13-bromo-quaterrylen-3,4-dicarbonsäureimid in 10 ml Toluol im Glove-Box zugegeben. Die Mischung wurde im Glove-Box auf 70°C erhitzt und 48 h bei dieser Temperatur gerührt. Nach Abkühlen auf Raumtemperatur wurde die organische Phase abgetrennt und das Lösungsmittel im Vakuum abgezogen. Das Rohprodukt wurde einer Säulenchromatographie an Kieselgel mit CH₂Cl₂ als Eluens unterzogen.

Es wurden 130 mg Produkt in Form eines schwarzgrünen Feststoffs erhalten, was einer Ausbeute von 83% entspricht.

Analytische Daten:
Rf = 0,68 (Toluol);
MS (Maldi): m/z (rel. Int.) = 2286.5(100%) [M+].
UV-Vis (Toluol): λₘₐₓ (ε): 753 (340000), 687 (181000), 359 (39000).

### Beispiel 5

### Herstellung von N-(2,6-Diisopropylphenyl-1,6,11,15-tetra[4-(1,1,3,3-tetramethylbutyl)-phenoxy]-13-(13-[N-(2,6-diisopropylphenyl)]-1,6,11,15-tetra[4-(1,1,3,3-tetramethylbutyl)-phenoxy]quaterrylen-3,4-dicarbonsäureimid)quaterrylen-3,4-dicarbonsäureimid

0,74 g (0,47 mmol) N-(2,6-Diisopropylphenyl-1,6,11,15-tetra[4-(1,1,3,3-tetramethylbutyl)-phenoxy]-13-cholro-quaterrylen-3,4-dicarbonsäureimid wurde unter N₂ in 10 ml Toluol und 1 ml Wasser bei Raumtemperatur gerührt. Nach 10 min gab man 0,14 g (0,56 mmol) Bispinacolato-diboron, 0,22 g (0,94 mmol) Kaliumphosphat-Monohydrat, 0,007g (0,017 mmol) 2-Dicyclohexylphosphino-2,6-dimethoxy-biphenyl und 0,002 g (0,008 mmol) Palladium-(II)-acetat hinzu. Die Mischung wurde auf 100°C erhitzt und 24 h bei dieser Temperatur gerührt. Das abgekühlte Reaktionsgemisch wurde mit 50 ml Toluol versetzt und mit 50 ml HCl (1 M) extrahiert. Die organische Phase wurde mit MgSO₄ getrocknet und eingedampft. Der Rückstand wurde einer Säulenchromatographie an Kieselgel mit Dichlormethan:n-Hexan (7:3) als Eluens unterzogen.

Es wurden 0,46 g Produkt in Form eines schwarzgrünen Feststoffs erhalten, was einer Ausbeute von 64% entspricht.

Analytische Daten:
UV-Vis (CH2Cl2): λₘₐₓ (ε) = 773 (200 000 M-1 cm-1);
MS (Maldi): m/z (rel. Int.) = 3089,4 (100%) [M-].

### Beispiel 6

### Cyclodehydrierung zum N,N'-Bis(2,6-diisopropylphenyl)-1,6,9,30-tetra(2,6-diisopropylphenylphenoxy)-12,17,22,27-tetra[4-(1,1,3,3-tetramethylbutyl)-phenoxy]-heptarylen-3,4:19,20-tetracarbonsäuredimid

Eine Mischung von 0,03 g (0,01 mmol) Produkt aus Beispiel 3, 0,063 g (0,5 mmol) Kaliumcarbonat und 10 mL Ethanolamin wurde auf 120°C unter Stickstoffatmosphare erhitzt. Nach 6 h war noch viel Edukt mittels DC beobachtet. Nach 30 h war Edukt komplett umgesetzt.

Nach Abkühlen wurde das Reaktionsprodukt auf Wasser gefällt, abfiltriert, mit heißem Wasser und abschließend mit Hexans gewaschen, bis der Ablauf farblos wurde. Dem Ruckstand wurde übernacht ein Soxhlet-Extraktion mit Hexans unterzogen. Das Produkt wurde im Vakuum bei 70°C getrocknet.

Es wurden 18 mg Produkt in Form eines schwarzen Feststoffs erhalten, was einer Ausbeute von 63% entspricht.

Analytische Daten:
UV-Vis (CHCl₃): λₘₐₓ = 973, 862 nm;
MS (Maldi): m/z (rel. Int.) = 2615,5 (100%) [M+].

### Beispiel 7

### Cyclodehydrierung zum N,N'-Bis(2,6-diisopropylphenyl)-1,6,11,14,19,24,29,32-octa[4-(1,1,3,3-tetramethylbutyl)phenoxy]octarylen-3,4:21,22-tetracarbonsäuredimid

Eine Mischung von 0,4 g (0,13 mmol) Produkt aus Beispiel 5, 0,54 g (1,4 mmol) Kaliumcarbonat, 10 mL Ethanolamin und 5 ml Diethylenglykoldiethylether wurde auf 120°C unter Stickstoffatmosphäre erhitzt. Die Reaktion wurde vom DC und UV-Vis Spektrum per Stunde beobachtet. Nach 3 Stunden war das Edukt komplett umgesetzt. Nach Abkühlen wurde das Reaktionsprodukt auf 1 M HCl gefällt, abfiltriert, mit heißem Wasser gewaschen, bis der Ablauf farblos wurde. Das Produkt wurde im Vakuum bei 70°C getrocknet.

Es wurden 0,4 g Produkt in Form eines schwarzen Feststoffs erhalten, was einer Ausbeute von 99% entspricht. Die Lösung in CH3Cl des Feststoffes ist fast farblos.

Analytische Daten:
UV-Vis (CH₂Cl₂): λₘₐₓ (ε) = 1066 nm (2620 000 M-1cm-1);
MS (Maldi): m/z (rel. Int.) = 3087,5 (100%) [M+].

## Patentansprüche

1. Verfahren zur Herstellung von Heptarylen- und Octarylentetracarbonsäurediimiden, die Schritte enthaltend
(a) Kupplung mindestens einer Quaterrylenverbindung der Formel (II) 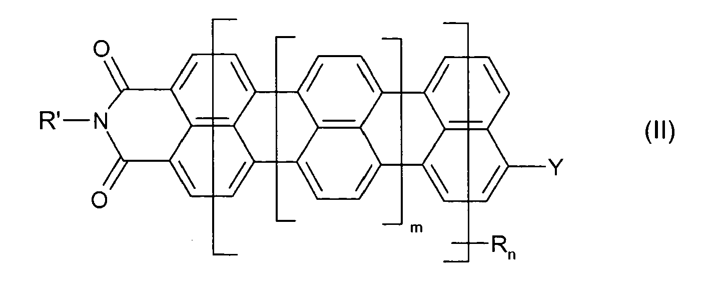 mit mindestens einer Verbindung der Formel (III) 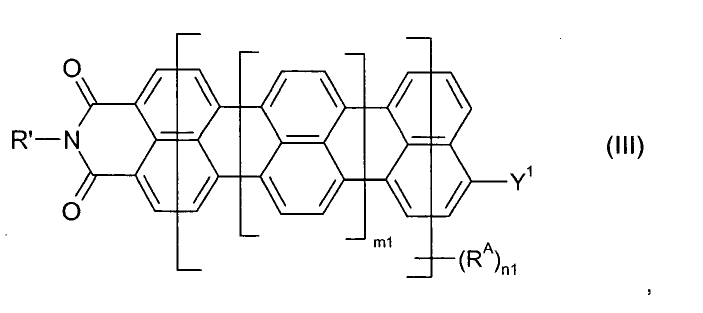 wobei
Y, Y¹ beide Halogen sind oder ein Rest von Y, Y¹ Halogen und der andere B(OR")₂ ist;
Jedes R, R^{A} unabhängig voneinander gleiche oder verschiedene der folgenden Reste sind:
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder - SO₂- unterbrochen sein kann und das ein- oder mehrfach substituiert sein kann durch: C₁-C₁₂-Alkoxy, C₁-C₆-Alkylthio, -C≡CR¹,
-CR¹=CR¹₂, Hydroxy, Mercapto, Halogen, Cyano, Nitro, -NR²R³,
-NR²COR³, -CONR²R³, -SO₂NR²R³, -COOR², -SO₃R², -PR²R³ ,-POR²R³, Aryl und/oder gesättigtes oder ungesättigtes C₄-C₇-Cycloalkyl, dessen Kohlenstoffgerüst durch eine oder mehrere Gruppierungen -O-, -S-,
-NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder - SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder - SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², - SO₃R², -PR²R³ und/oder -POR²R³;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, - CONR²R³, -SO₂NR²R³, -COOR², -SO₃R², -PR²R³ und/oder -POR²R³ substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, - COOR², -SO₃R², -PR²R³ und/oder -POR²R³;
jedes R' jeweils unabhängig Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder - SO₂- unterbrochen sein kann und das ein- oder mehrfach durch die als Substituenten für die Reste R genannten Reste (ii), (iii), (iv) und/oder (v) substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem durch die als Substituenten für die Reste R genannten Reste (i), (ii), (iii), (iv) und/oder (v) substituiert sein kann; oder
Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem substituiert sein kann durch die als Substituenten für die Reste R genannten Reste (i), (ii), (iii), (iv), (v), Aryl- und/oder Hetarylazo, das jeweils durch C₁-C₁₀-Alkyl, C₁-C₆-Alkoxy und/oder Cyano substituiert sein kann, ist;
jedes R" unabhängig voneinander Wasserstoff, C₁-C₃₀-Alkyl, C₅-C₈-Cycloalkyl, Aryl oder Hetaryl ist oder miteinander verbunden unter Ausbildung eines die beiden Sauerstoffatome sowie das Boratom enthaltenden 5- bis 7-gliedrigen Rings, an den ungesättigte oder gesättigte Ringe anneliert sein können und der an den Kohlenstoffatomen durch bis zu 4 C₁-C₃₀-Alkyl-, C₅-C₈-Cycloalkyl-, Aryl- oder Hetarylgruppen substituiert sein kann, sind;
R¹ Wasserstoff oder C₁-C₁₈-Alkyl, wobei die Reste R¹ gleich oder verschieden sein können, wenn sie mehrfach auftreten, ist;
R², R³ unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder-COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, sind;
und wobei m, m1, n, n1 mindestens eine der folgenden Bedingungen erfüllen:
m = 2, m1 = 1 und n, n1 sind ganze Zahlen, deren Summe eine Zahl von 0 bis 12 ergibt;
m = 2, m1 = 2 und n, n1 sind ganze Zahlen, deren Summe eine Zahl von 0 bis 16 ergibt;
(b) Cyclodehydrierung des in Schritt (a) erhaltenen Umsetzungsproduktes zu einer Rylenverbindung der allgemeinen Formel (I) 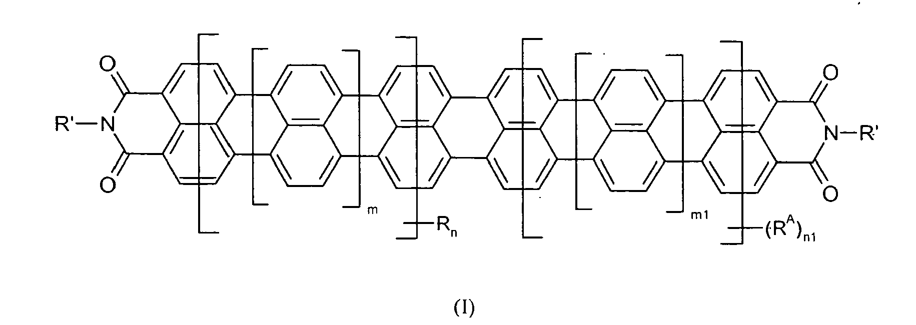 oder deren Mischungen.

2. Heptarylentetracarbonsäurediimid der allgemeinen Formel (I) oder Mischungen davon, wobei n und n1 ganze Zahlen sind, deren Summe eine Zahl von 0 bis 12 ergibt und R, R^{A}, R', m, m1 die in Anspruch 1 angegebene Bedeutung besitzen.

3. Octarylentetracarbonsäurediimid der allgemeinen Formel (I) oder Mischungen davon, wobei n und n1 ganze Zahlen sind, deren Summe eine Zahl von 0 bis 16 ergibt und R, R^{A}, R', m, m1 die in Anspruch 1 angegebene Bedeutung besitzen.

4. Diimid oder Mischungen davon nach Anspruch 2 oder 3, **dadurch gekennzeichnet, dass** R und R^{A} die gleiche Bedeutung besitzen.

5. Diimid oder Mischungen davon nach einem der Ansprüche 2 bis 4, **dadurch gekennzeichnet, dass** R, R^{A} unabhängig voneinander Aryloxy oder Arylthio sind, wobei das gesamte Ringsystem ein- oder mehrfach durch die Reste (i), (ii), (iii), (iv) und/oder (v) wie in Anspruch 1 angegeben, substituiert sein kann.

6. Diimid oder Mischungen davon nach Anspruch 5, **dadurch gekennzeichnet, dass** R, R^{A} unabhängig voneinander ein- oder mehrfach durch einen Rest (i) substituiert sein kann.

7. Diimid oder Mischungen davon nach Anspruch 5 oder 6, **dadurch gekennzeichnet, dass** R, R^{A} unabhängig voneinander 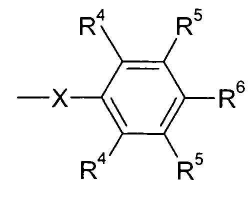 sind,
wobei
- X O oder S ist und
R⁴, R⁵, R⁶ unabhängig voneinander Wasserstoff oder die Reste (i), (ii), (iii), (iv) und/oder (v) wie in Anspruch 1 angegeben mit der Maßgabe sein können, dass zumindest einer der Reste R⁴, R⁶ kein Wasserstoff ist.

8. Diimid nach Anspruch 7, **dadurch gekennzeichnet, dass** wenn R⁴ C₁-C₃₀ Alkyl oder C₃-C₈ Cycloalkyl ist, in der 1-Position kein ternäres Kohlenstoffatom auftritt.

9. Diimid oder Mischungen davon nach Anspruch 7 oder 8, **dadurch gekennzeichnet, dass** beide R⁴ kein Wasserstoff und R⁵, R⁶ Wasserstoff sind oder dass R⁶ kein Wasserstoff ist und R⁴, R⁵ Wasserstoff sind.

10. Diimid oder Mischungen davon nach einem der Ansprüche 2 bis 9, **dadurch gekennzeichnet, dass** jedes R' unabhängig voneinander C₁-C₃₀ Alkyl oder Aryl ist, wobei das gesamte Ringsystem ein- oder mehrfach durch die Reste (i), (ii), (iii), (iv) und/oder (v) wie in Anspruch 1 angegeben, substituiert sein kann.

11. Diimid oder Mischungen davon nach Anspruch 10, **dadurch gekennzeichnet, dass** R' ein- oder mehrfach durch einen Rest (i) substituiert ist.

12. Verwendung eines Diimids oder Mischungen davon nach einem der Ansprüche 2 bis 11 zur Einfärbung von hochmolekularen organischen und anorganischen Materialien, als Dispergierhilfsmittel und Pigmentadditive für organische Pigmente, zur Herstellung im nahinfraroten Bereich des elektromagnetischen Spektrums absorbierender wässriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-laserstrahlenabsorbierende Materialien beim Schweißbehandeln von Kunststoffteilen, als Halbleiter in der organischen Elektronik, als Emitter in Elektro- und Chemilumineszenzanwendungen oder als Aktivkomponente in der Photovoltaik.

13. Verfahren zur Herstellung einer Quaterrylenverbindung der allgemeinen Formel (II) 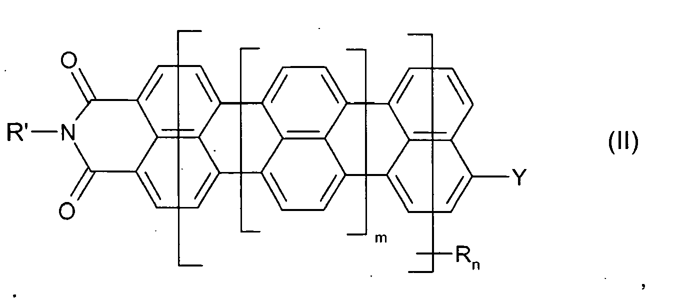 wobei Y gleich Halogen und R', R, n, m die in Anspruch 1 angegebene Bedeutung besitzen, die Schritte enthaltend
(a) Kupplung mindestens einer Perylenverbindung der Formel (II) 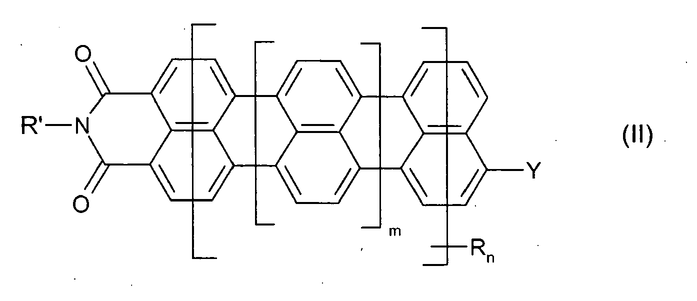 mit mindestens einer Verbindung der Formel (IIIa) und/oder (IIIa') 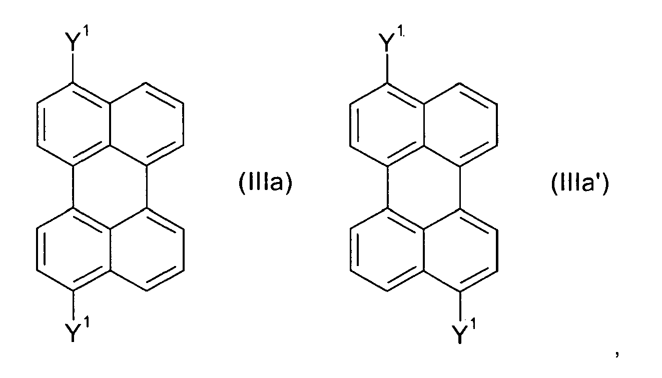 wobei
ein Rest von Y, Y¹ Halogen und der andere B(OR")₂ ist, m = 0 ist und R, R', n, die in Anspruch 1 angegebene Bedeutung besitzen, zu einer Verbindung der allgemeinen Formel (IIIb)/(IIIb') 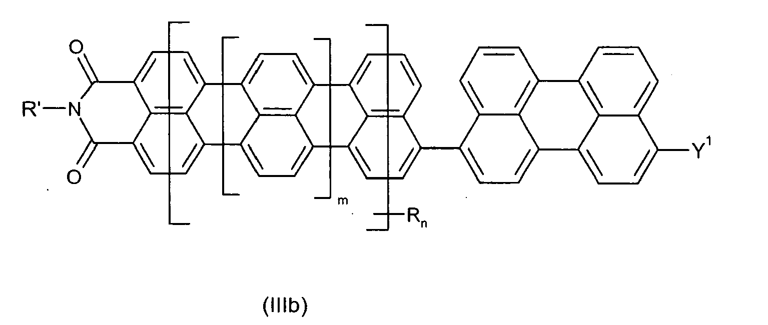 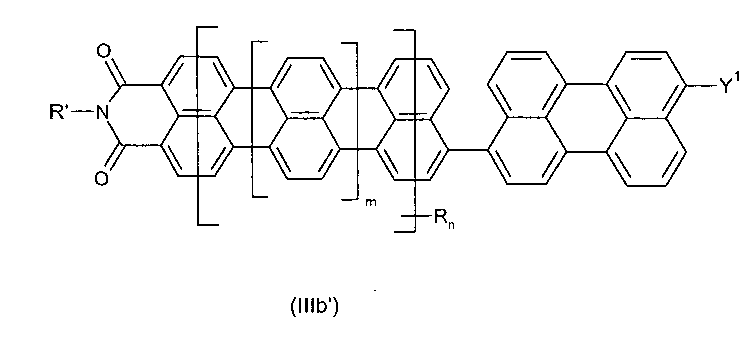 und
(b) Cyclodehydrierung der in Schritt (a) erhaltenen Verbindung der allgemeinen Formel (IIIb)/(IIIb') zu einer Rylenverbindung der allgemeinen Formel (II), wobei Y¹ in Formel (IIIb)/(IIIb') die Bedeutung von Y in Formel (II) hat.

14. Verbindung der allgemeinen Formel (IIIb) oder (IIIb') 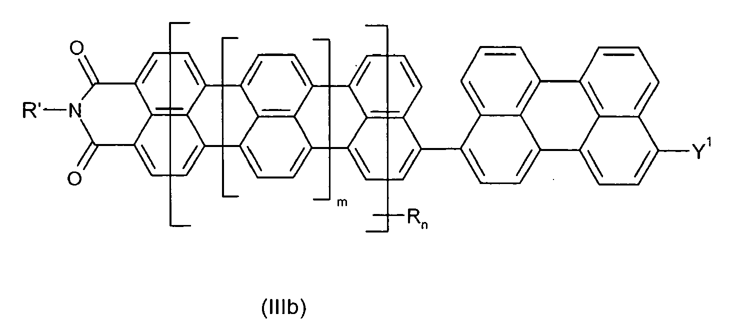 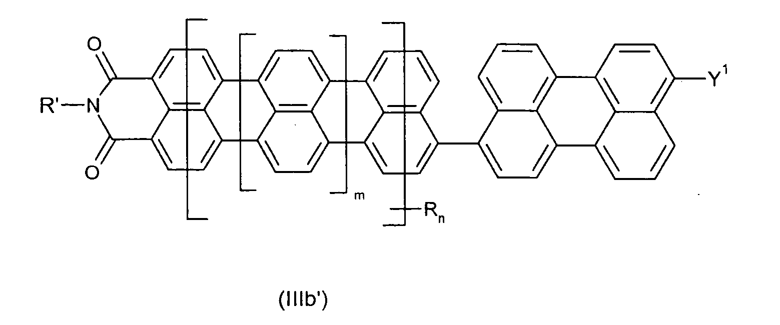 oder Mischungen davon, wobei R, R', n, m, Y¹ die in Anspruch 13 angegebene Bedeutung besitzen.

## Claims

1. A process for preparing heptarylene- and octarylenetetracarboximides, comprising the steps of
(a) coupling at least one quaterrylene compound of the formula (II) 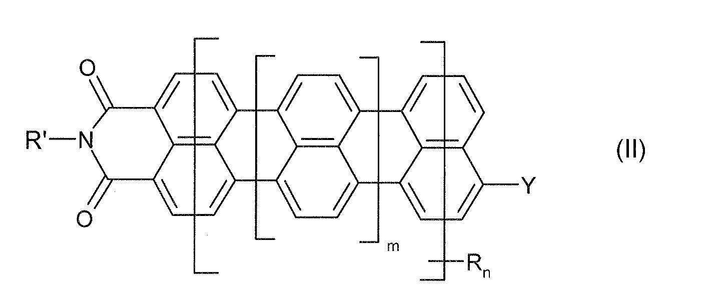 with at least one compound of the formula (III) 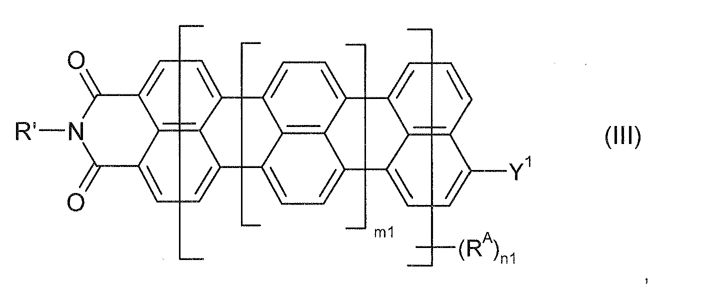 where
Y, Y¹ are each halogen, or one radical of Y, Y¹ is halogen and the other is B(OR")₂;
each R, R^{A} are independently identical or different radicals selected from the following:
aryloxy, arylthio, hetaryloxy or hetarylthio, to each of which may be fused further saturated or unsaturated 5- to 7-membered rings whose carbon skeleton may be interrupted by one or more -O-, -S-, -NR¹-, - N=CR¹-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂-moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³, -POR²R³, aryl and/or saturated or unsaturated C₄-C₇-cycloalkyl whose carbon skeleton may be interrupted by one or more -O-, -S-, -NR¹-, -N=CR¹-, -CR¹=CR¹-, -CO-, -SO- and/or -SO₂- moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹-, -N=CR¹-, -CR¹=CR¹-, -CO-, -SO- and/or -SO₂-moieties and to which may be fused further saturated or unsaturated 5-to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³ and/or -POR²R³;
(iii) aryl or hetaryl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³, -POR²R³, aryl and/or hetaryl, each of which may be substituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR², -SO₃R², -PR²R³ and/or -POR²R³;
(iv) a -U-aryl radical which may be mono- or polysubstituted by the above radicals specified as substituents for the aryl radicals (iii), where U is a -O-, -S-, -NR¹-, -CO-, -SO- or -SO₂- moiety;
(v) C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³ and/or -POR²R³;
each R' is independently hydrogen;
C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by the (ii), (iii), (iv) and/or (v) radicals specified as substituents for the R radicals;
C₃-C₈-cycloalkyl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be substituted by the (i), (ii), (iii), (iv) and/or (v) radicals specified as substituents for the R radicals; or
aryl or hetaryl to which may be fused further saturated or unsaturated 5-to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be substituted by the (i), (ii), (iii), (iv), (v) radicals specified as substituents for the R radicals, aryl-and/or hetarylazo, each of which may be substituted by C₁-C₁₀-alkyl, C₁-C₆-alkoxy and/or cyano;
each R" is independently hydrogen, C₁-C₃₀-alkyl, C₅-C₈-cycloalkyl, aryl or hetaryl or both are joined together with formation of a 5- to 7-membered ring which comprises the two oxygen atoms and also the boron atom, to which may be fused unsaturated or saturated rings and which may be substituted on the carbon atoms by up to 4 C₁-C₃₀-alkyl, C₅-C₈-cycloalkyl, aryl or hetaryl groups;
R¹ is hydrogen or C₁-C₁₈-alkyl, where the R¹ radicals may be the same or different when they occur more than once;
R²,R³ are each independently hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR¹;
aryl or hetaryl to each of which may be fused further saturated or unsaturated 5- to 7-membered rings whose carbon skeleton may be interrupted by one or more -O-, -S-, -CO- and/or -SO₂- moieties, where the entire ring system may be mono- or polysubstituted by C₁-C₁₂-alkyl and/or the above radicals specified as substituents for alkyl;
and where m, m1, n, n1 fulfill at least one of the following conditions:
m = 2, m1 = 1 and n, n1 are integers whose sum adds up to from 0 to 12;
m = 2, m1 = 2 and n, n1 are integers whose sum adds up to from 0 to 16;
(b) cyclodehydrogenating the reaction product obtained in step (a) to give a rylene compound of the general formula (I) 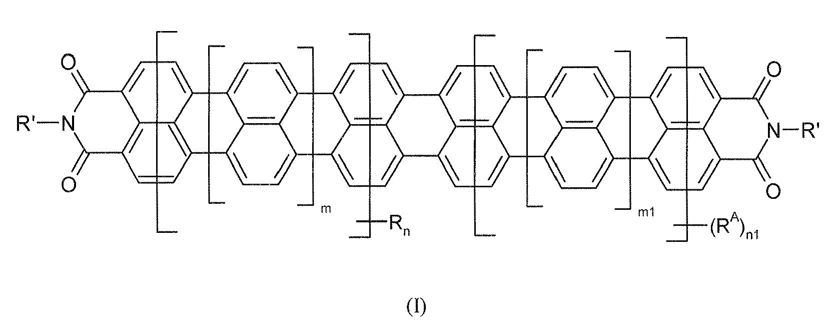 or mixtures thereof.

2. The heptarylenetetracarboximide of the general formula (I) or mixtures thereof, where n and n1 are integers whose sum adds up to from 0 to 12 and R, R^{A}, R', m, m1 are each as defined in claim 1.

3. The octarylenetetracarboximide of the general formula (I) or mixtures thereof, where n and n1 are integers whose sum adds up to from 0 to 16 and R, R^{A}, R', m, m1 are each as defined in claim 1.

4. The diimide or mixtures thereof according to claim 2 or 3, wherein R and R^{A} are the same.

5. The diimide or mixtures thereof according to any of claims 2 to 4, wherein R, R^{A} are each independently aryloxy or arylthio, where the entire ring system may be mono- or polysubstituted by the (i), (ii), (iii), (iv) and/or (v) radicals as specified in claim 1.

6. The diimide or mixtures thereof according to claim 5, wherein R, R^{A} may each independently be mono- or polysubstituted by a (i) radical.

7. The diimide or mixtures thereof according to claim 5 or 6, wherein R, R^{A} are each independently 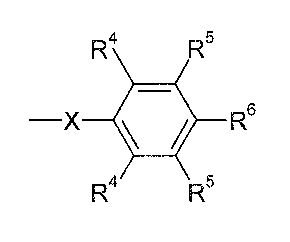 where
X is O or Sand
R⁴, R⁵, R⁶ may each independently be hydrogen or the (i), (ii), (iii), (iv) and/or (v) radicals as specified in claim 1, with the proviso that at least one of the R⁴, R⁶ radicals is not hydrogen.

8. The diimide according to claim 7, wherein, when R⁴ is C₁-C₃₀-alkyl or C₃-C₈-cycloalkyl, a ternary carbon atom does not occur in the 1-position.

9. The diimide or mixtures thereof according to claim 7 or 8, wherein neither R⁴ is hydrogen and R⁵, R⁶ are each hydrogen, or R⁶ is not hydrogen and R⁴, R⁵ are each hydrogen.

10. The diimide or mixtures thereof according to any of claims 2 to 9, wherein each R' is independently C₁-C₃₀-alkyl or aryl, where the entire ring system may be mono- or polysubstituted by the (i), (ii), (iii), (iv) and/or (v) radicals as specified in claim 1.

11. The diimide or mixtures thereof according to claim 10, wherein R' is mono- or polysubstituted by a (i) radical.

12. The use of a diimide or mixtures thereof according to any of claims 2 to 11 for coloring high molecular weight organic and inorganic materials, as dispersing assistants and pigment additives for organic pigments, for preparing aqueous polymer dispersions which absorb in the near infrared region of the electromagnetic spectrum, for obtaining markings and inscriptions which absorb infrared light and are invisible to the human eye, as infrared absorbers for heat management, as IR laser beam-absorbing materials in the fusion treatment of plastics parts, as semiconductors in organic electronics, as emitters in electro- and chemiluminescence applications, or as active components in photovoltaics.

13. A process for preparing a quaterrylene compound of the general formula (II) 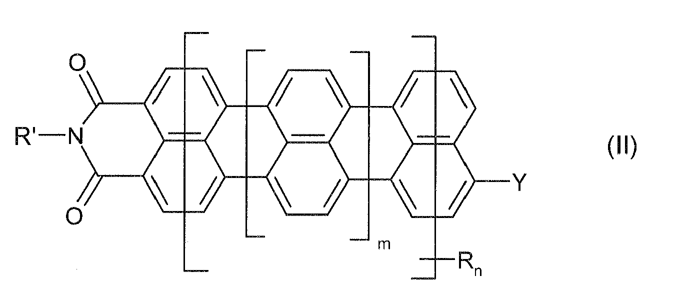 where Y is halogen and R', R, n, m are each as defined in claim 1, comprising the steps of
(a) coupling at least one perylene compound of the formula (II) 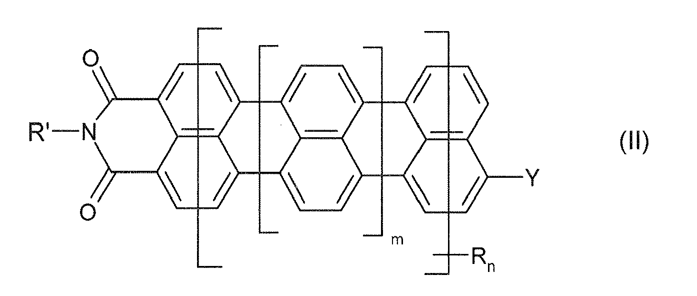 with at least one compound of the formula (IIIa) and/or (IIIa') 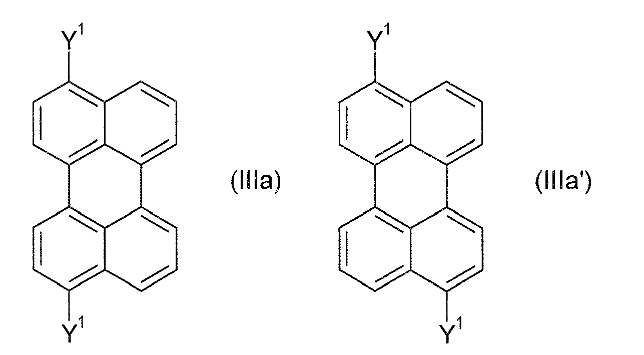 where
one radical of Y, Y¹ is halogen and the other is B(OR")₂, m = 0, and R, R', n are each as defined in claim 1 to give a compound of the general formula (IIIb)/(IIIb') 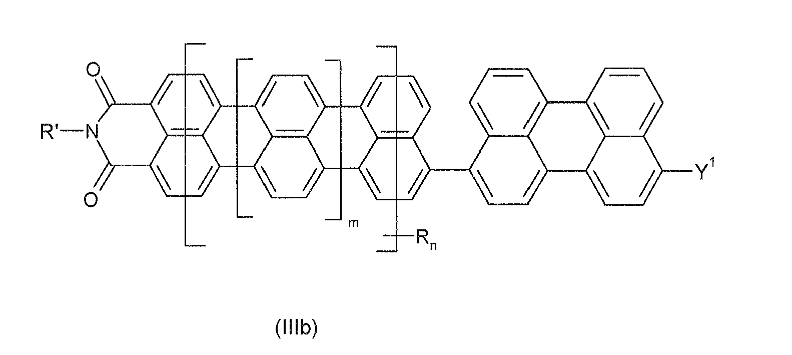 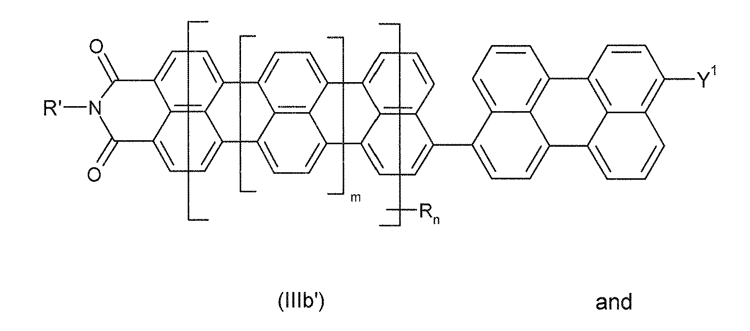
(b) cyclodehydrogenating the compound of the general formula (IIIb)/(IIIb') obtained in step (a) to give arylene compound of the general formula (II), where Y¹ in formula (IIIb)/(IIIb') is as defined for Y in formula (II).

14. A compound of the general formula (IIIb) or (IIIb') 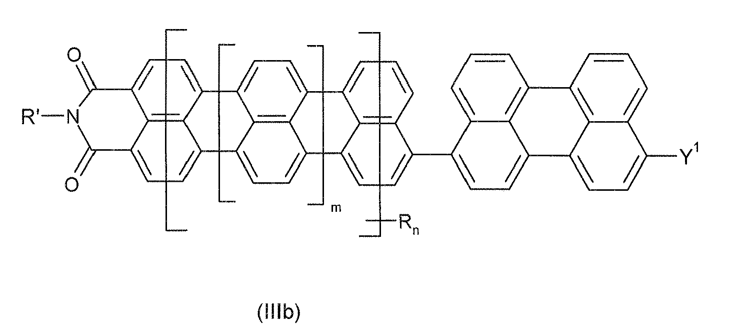 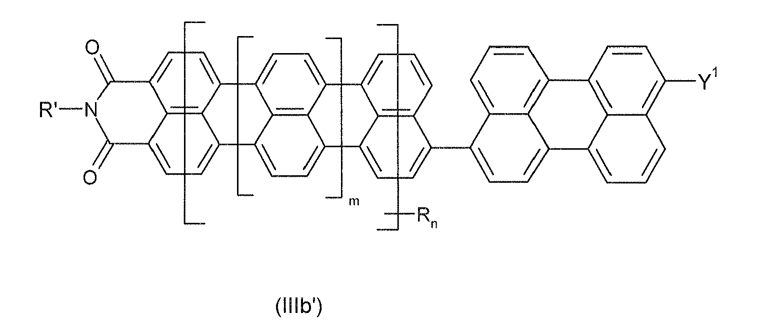 or mixtures thereof, where R, R', n, m, Y¹ are each as defined in claim 13.

## Revendications

1. Procédé pour la préparation de diimides de l'acide heptarylènetétracarboxylique et octarylènetétracarboxylique, comprenant les étapes de (a) couplage d'au moins un composé quater-rylène de
formule (II) 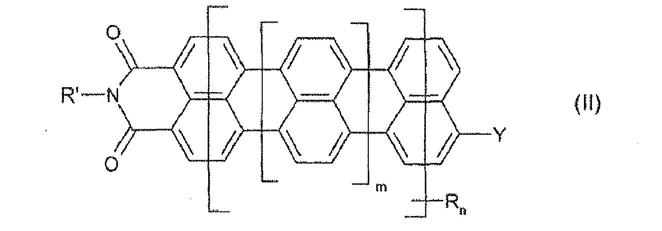 avec au moins un composé de formule (III) 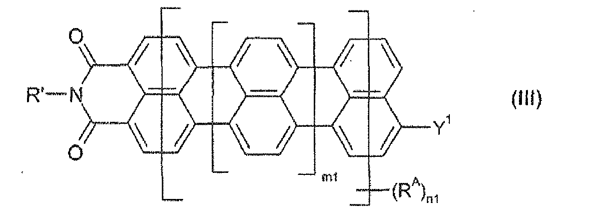 où
Y, Y¹ représentent tous deux halogène ou un radical parmi Y, Y¹ représente halogène et l'autre représente B(OR")₂ ;
chaque R, R^{A} représente, indépendamment l'un de l'autre, des radicaux identiques ou différents parmi les radicaux suivants :
aryloxy, arylthio, hétaryloxy ou hétarylthio, sur lequel à chaque fois d'autres cycles saturés ou insaturés de 5 à 7 chaînons, dont la structure carbonée peut être interrompue par un ou plusieurs groupements -O-, -S-, -NR¹-, -N=CR¹-, -CO-, -SO- et/ou -SO₂-, peuvent être condensés, l'ensemble du système cyclique pouvant être monosubstitué ou polysubstitué par les radicaux (i), (ii), (iii), (iv) et/ou (v) :
(i) C₁-C₃₀-alkyle, dont la chaîne carbonée peut être interrompue par un ou plusieurs groupements -O-, -S-, -NR¹-, -N=CR¹-, -C≡C-, -CR¹≡CR¹-, -CO-, -SO- et/ou -SO₂- et qui peut être monosubstitué ou polysubstitué par : C₁-C₁₂-alcoxy, C₁-C₆-alkylthio, -C≡CR¹,
-CR¹=CR¹₂, hydroxy, mercapto, halogène, cyano, nitro, -NR²R³,
-NR²COR³, -CONR²R³, -SO₂NR²R³, -COOR², -SO₃R², -PR²R³, -POR²R³, aryle et/ou C₄-C₇-cycloalkyle saturé ou insaturé, dont la structure carbonée peut être interrompue par un ou plusieurs groupements -O-, -S-, -NR¹-, -N=CR¹, -CR¹=CR¹-, -CO-, -SO- et/ou -SO₂-, les radicaux aryle et cycloalkyle pouvant à chaque fois être monosubstitués ou polysubstitués par C₁-C₁₈-alkyle et/ou les radicaux ci-dessus mentionnés comme substituants pour alkyle ;
(ii) C₃-C₈-cycloalkyle, dont la structure carbonée peut être interrompue par un ou plusieurs groupements -O-, -S-, -NR¹-, -N=CR¹-, -CR¹=CR¹-, -CO-, -SO- et/ou -SO₂- et sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², - SO₃R², -PR²R³ et/ou -POR²R³ ;
(iii) aryle ou hétaryle, sur lequel d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³, -POR²R³, aryle et/ou hétaryle, qui peut être substitué à chaque fois par C₁-C₁₈-alkyle, C₁-C₁₂-alcoxy, hydroxy, mercapto, halogène, cyano, nitro, -NR²R³, -NR²COR³, -CONR²R³, -SO₂NR²R³, -COOR², -SO₃R², -PR²R³ et/ou -POR²R³ ;
(iv) un radical -U-aryle, qui peut être monosubstitué ou polysubstitué par les radicaux ci-dessus, mentionnés comme substituants pour le radical aryle (iii), où U signifie un groupement -O-, -S-, -NR¹-, -CO-, -SO- ou -SO₂- ;
(v) C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³ et/ou -POR²R³ ;
chaque R¹ représente à chaque fois indépendamment l'un de l'autre hydrogène ;
C₁-C₃₀-alkyle, dont la chaîne carbonée peut être interrompue par un ou plusieurs groupements -O-, -S-, -NR¹-, -N=CR¹-, -C≡CR-, -CR¹=CR¹-, -CO-, -SO- et/ou -SO₂- et qui peut être substitué une ou plusieurs fois par les radicaux mentionnés comme substituants pour les radicaux R (ii), (iii), (iv) et/ou (v) ;
C₃-C₈-cycloalkyle, sur lequel d'autres cycles saturés ou insaturés, de 5 à 7 chaînons, dont la structure carbonée peut être substituée par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être substitué par les radicaux (i), (ii), (iii), (iv) et/ou (v) mentionnés comme substituants pour les radicaux R ; ou
aryle ou hétaryle, sur lequel d'autres cycles saturés ou insaturés, de 5 à 7 chaînons, dont la structure carbonée peut être substituée par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être substitué par les radicaux (i), (ii), (iii), (iv) et/ou (v) mentionnés comme substituants pour les radicaux R, arylazo et/ou hétarylazo, qui peut être à chaque fois substitué par C₁-C₁₀-alkyle, C₁-C₆-alcoxy et/ou cyano ;
chaque R" représente, indépendamment l'un de l'autre, hydrogène, C₁-C₃₀-alkyle, C₅-C₈-cycloalkyle, aryle ou hétaryle ou ils forment, en étant reliés l'un avec l'autre, un cycle de 5 à 7 chaînons, contenant les deux atomes d'oxygène ainsi que l'atome de bore, sur lequel des cycles insaturés ou saturés peuvent être condensés et qui peut être substitué, sur les atomes de carbone,
par jusqu'à 4 groupes C₁-C₃₀-alkyle, C₅-c₈-cycloalkyle, aryle ou hétaryle ;
R¹ représente hydrogène ou C₁-C₁₈-alkyle, où les radicaux R¹ peuvent être identiques ou différents, lorsqu'ils existent plusieurs fois ;
R², R³ représentent, indépendamment l'un de l'autre, hydrogène ;
C₁-C₁₈-alkyle, dont la chaîne carbonée peut être interrompe par un ou plusieurs groupements -O-, -S-, -CO-, -SO- et/ou -SO₂- et qui peut être monosubstitué ou polysubstitué par C₁-C₁₂-alcoxy, C₁-C₆-alkylthio, hydroxy, mercapto, halogène, cyano, nitro et/ou -COOR¹ ;
aryle ou hétaryle, sur lequel à chaque fois d'autres cycles saturés ou insaturés, de 5 à 7 chaînons, dont la structure carbonée peut être substituée par un ou plusieurs groupements -O-, -S-, -CO- et/ou -SO₂-, peuvent être condensés, l'ensemble du système cyclique pouvant être monosubstitué ou polysubstitué par C₁-C₁₂-alkyle et/ou par les radicaux ci-dessus, mentionnés comme substituants alkyle ;
et où m, m1, n, n1 remplissent au moins une des conditions suivantes :
m = 2, m1 = 1 et n, n1 sont des nombres entiers, dont la somme vaut un nombre de 0 à 12 ;
m = 2, m1 = 2 et n, n1 sont des nombres entiers, dont la somme vaut un nombre de 0 à 16 ;
(b) cyclodéshydrogénation du produit de transformation obtenu dans l'étape (a) en un composé rylène de formule générale (I) 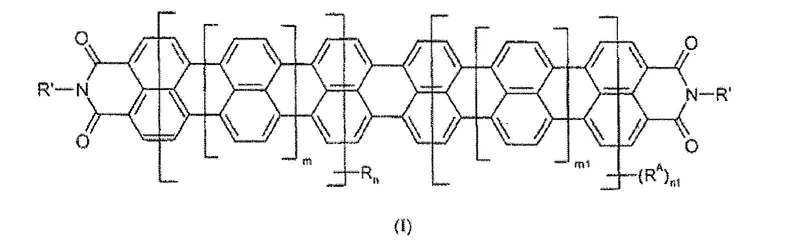 ou leurs mélanges.

2. Diimide de l'acide heptarylènetétracarboxylique de formule générale (I) ou mélanges de celui-ci, où n et n1 valent des nombres entiers, dont la somme vaut un nombre de 0 à 12 et R, R^{A}, R', m, m1 présentent la signification indiquée dans la revendication 1.

3. Diimide de l'acide octarylènetétracarboxylique de formule générale (I) ou mélanges de celui-ci, où n et n1 valent des nombres entiers, dont la somme vaut un nombre de 0 à 16 et R, R^{A}, R', m, m1 présentent la signification indiquée dans la revendication 1.

4. Diimide ou mélanges de celui-ci selon la revendication 2 ou 3, **caractérisé en ce que** R et R^{A} présentent la même signification.

5. Diimide ou mélanges de celui-ci selon l'une quelconque des revendications 2 à 4, **caractérisé en ce que** R, R^{A} représentent, indépendamment l'un de l'autre, aryloxy ou arylthio, où l'ensemble du système cyclique peut être monosubstitué ou polysubstitué par les radicaux (i), (ii), (iii), (iv) et/ou (v), comme indiqué dans la revendication 1.

6. Diimide ou mélanges de celui-ci selon la revendication 5, **caractérisé en ce que** R, R^{A} indépendamment l'un de l'autre, peuvent être monosubstitués ou polysubstitués par un radical (i).

7. Diimide ou mélanges de celui-ci selon la revendication 5 ou 6, **caractérisé en ce que** R, R^{A} représentent, indépendamment l'un de l'autre, 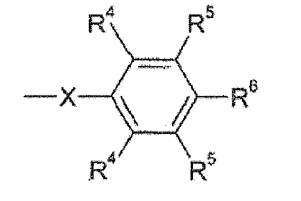 où
X représente O ou S et
R⁴, R⁵, R⁶ représentent, indépendamment l'un de l'autre, hydrogène ou les radicaux (i), (ii), (iii), (iv) et/ou (v) comme indiqués dans la revendication 1 à condition qu'au moins un des radicaux R⁴, R⁶ ne représente pas hydrogène.

8. Diimide selon la revendication 7, **caractérisé en ce que**, lorsque R⁴ représente C₁-C₃₀-alkyle ou C₃-C₈-cycloalkyle, il n'existe pas d'atome de carbone ternaire dans la 1ère position.

9. Diimide ou mélanges de celui-ci selon la revendication 7 ou 8, **caractérisé en ce que** les deux radicaux R⁴ ne représentent pas hydrogène et R⁵, R⁶ représentent hydrogène ou **en ce que** R⁶ ne représente pas hydrogène et R⁴, R⁵ représentent hydrogène.

10. Diimide ou mélanges de celui-ci selon l'une quelconque des revendications 2 à 9, **caractérisé en ce que** chaque R' représente, indépendamment l'un de l'autre, C₁-C₃₀-alkyle ou aryle, où l'ensemble du système cyclique peut être monosubstitué ou polysubstitué par les radicaux (i), (ii), (iii), (iv) et/ou (v), comme indiqué dans la revendication 1.

11. Diimide ou mélanges de celui-ci selon la revendication 10, **caractérisé en ce que** R' est monosubstitué ou polysubstitué par un radical (i).

12. Utilisation d'un diimide ou de mélanges de celui-ci selon l'une quelconque des revendications 2 à 11 pour la coloration de matériaux organiques et inorganiques de haut poids moléculaire, comme adjuvant de dispersion et additif pigmentaire pour pigments organiques, pour la préparation de dispersions polymères aqueuses absorbant dans la plage infrarouge proche du spectre électromagnétique, afin d'obtenir des marquages et des inscriptions invisibles pour l'oeil humain, absorbant la lumière infrarouge, comme absorbants d'infrarouges pour la gestion de chaleur, comme matériaux absorbant un rayon laser IR lors du traitement par soudure de pièces en matériau synthétique, comme semi-conduction dans l'électronique organique, comme émetteur dans des utilisations électriques et chimiluminescentes ou comme composant actif dans la photovoltaïque.

13. Procédé pour la préparation d'un composé quater-rylène de formule générale (II), 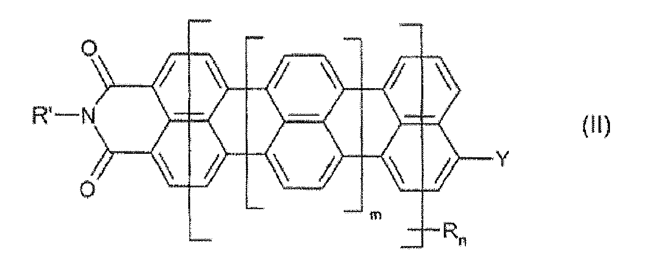 où Y représente halogène et R', R, n, m présentent la signification indiquée dans la revendication 1, comprenant les étapes
(a) couplage d'au moins un composé pérylène de formule (II) 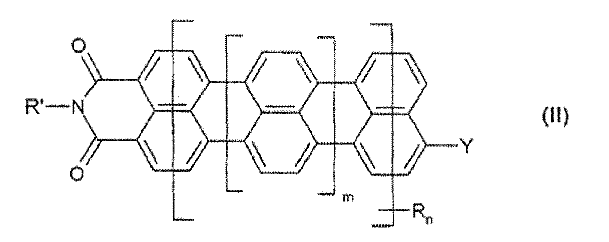 avec au moins un composé de formule (IIIa) et/ou (IIIa') 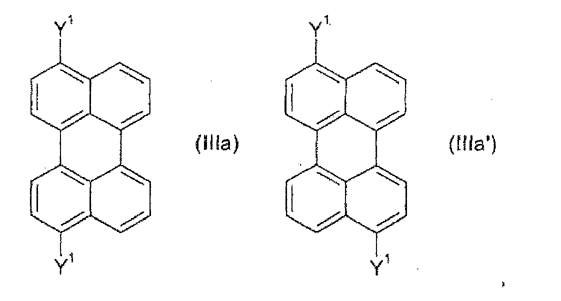 où un radical parmi Y, Y¹ représente halogène et l'autre représente B(OR")₂, m = 0 et R, R', n présentent la signification indiquée dans la revendication 1, en un composé de formule générale (IIIb)/(IIIb') 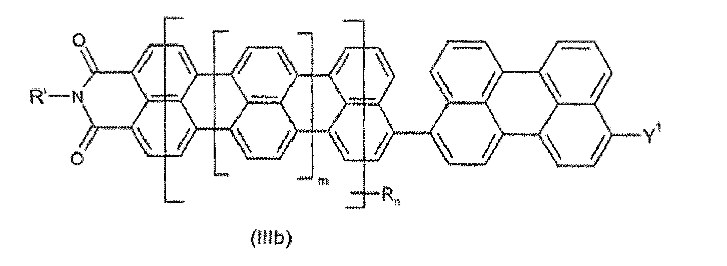 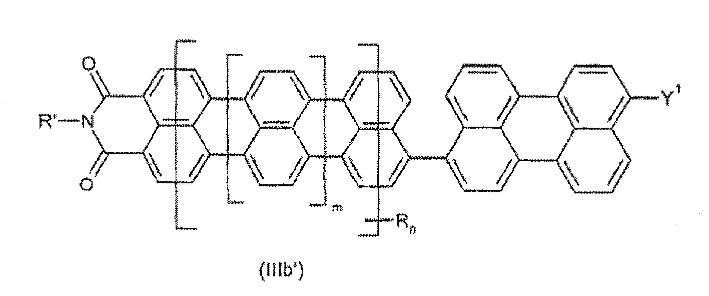 et
(b) cyclodéshydrogénation du composé obtenu dans l'étape (a) de formule générale (IIIb)/(IIIb') en un composé rylène de formule générale (II), où Y¹ dans la formule (IIIb)/(IIIb') présente la signification de Y dans la formule (II).

14. Composé de formule générale (IIIb) ou (IIIb') 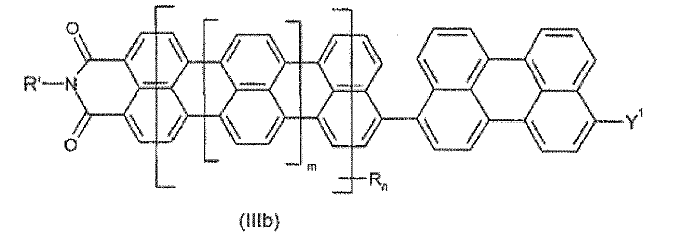 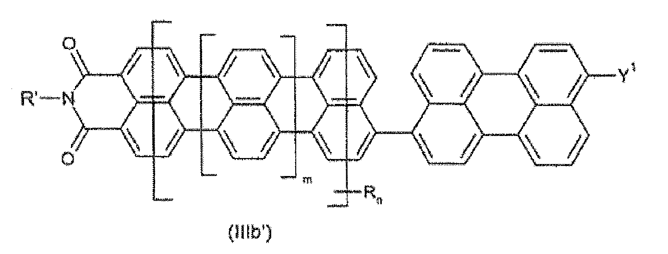 ou mélanges de celui-ci, où R, R', n, m, Y¹ présentent la signification indiquée dans la revendication 13.
